# EUROPEAN PATENT SPECIFICATION

(11) **EP 2 410 052 B1**
(45) Date of publication and mention of the grant of the patent: **02.12.2015**
(21) Application number: 10753741.7
(22) Date of filing: 17.03.2010
(51) Int. Cl.: C12Q 1/68

(54) **IN VITRO METHOD FOR THE DETECTION OF CANDIDA GLABRATA, DIAGNOSTIC KIT AND USE THEREOF**
IN-VITRO-VERFAHREN ZUR ERKENNUNG VON CANDIDA GLABRATA, DIAGNOSEKIT UND VERWENDUNG DAVON
PROCÉDÉ IN VITRO DE DÉTECTION DE CANDIDA GLABRATA, NÉCESSAIRE DE DIAGNOSTIC ET LEURS UTILISATIONS

(30) Priority: 18.03.2009 MX 2009002935
(43) Date of publication of application: 25.01.2012
(73) Proprietor: Instituto Potosino de Investigación Cientifica y Tecnológica, A.C., C.P. 78216 San Luis Posoti, S.L.P. (MX)
(72) Inventor: CASTAÑO NAVARRO, Irene Beatriz, C.P. 78210 San Luis Potosí, S.L.P. (MX); CUELLAR CRUZ, Mayra, C.P. 78210 San Luis Potosí, S.L.P. (MX); DE LAS PEÑAS NAVAS, Alejandro, C.P. 78210 San Luis Potosí, S.L.P. (MX)
(74) Representative: Jeannet, Olivier
(86) International application number: PCT/MX2010/000020
(87) International publication number: WO 2010/107292

(56) References cited:
- WO-A1-98/11257
- WO-A2-00/73499
- WO-A2-2006/059228
- DATABASE ENA [Online] 19 September 2003 (2003-09-19), "Candida glabrata Isc1p (ISC1), Hyr1p (HYR1), Epa1p (EPA1), Epa2p (EPA2), and Epa3p (EPA3) genes, complete cds.", XP002696387, retrieved from EBI Database accession no. AY344226
- DATABASE ENA [Online] 19 September 2003 (2003-09-19), "Candida glabrata Epa5p (EPA5) and Epa4p (EPA4) genes complete cds.", XP002696388, retrieved from EBI Database accession no. AY344225
- DATABASE ENA [Online] 7 July 2004 (2004-07-07), "Candida glabrata epithelial adhesin 7 (EPA7) gene, complete cds.", XP002696389, retrieved from EBI Database accession no. AY646926
- DATABASE ENA [Online] 7 July 2004 (2004-07-07), "Candida glabrata cell wall mannoprotein (DAN1) and epithelial adhesin 6 (EPA6) genes, complete cds.", XP002696390, retrieved from EBI Database accession no. AY646925
- CORMACK B P ET AL: "An adhesin of the yeast pathogen Candida glabrata mediating adherence to human epithelial cells", SCIENCE, vol. 285, no. 5427, 23 July 1999 (1999-07-23), pages 578-582, XP002385470, AMERICAN ASSOCIATION FOR THE ADVANCEMENT OF SCIENCE, WASHINGTON, DC; US ISSN: 0036-8075, DOI: 10.1126/SCIENCE.285.5427.578
- ROSAS-HERNÁNDEZ L. L. ET AL.: 'yKu70/yKu80 and Rifl regulate silencing differentially at telomeres in Candida glabrata' EUKARYOTIC CELL. vol. 7, no. 12, December 2008, pages 2168 - 2178, XP055103899
- CASTYEAR I. ET AL.: 'Telomere length control and transcriptional regulation of subtelomeric adhesins in Candida glabrata' MOLECULAR MICROBIOLOGY vol. 55, no. 4, 2005, pages 1246 - 1258, XP055104002
- DE LAS PENAS A. ET AL.: 'Virulence-related surface glycoproteins in the yeast pathogen Candida glabrata are encoded in subtelomeric clusters and subject to RAP1- and SIR-dependent transcriptional silencing' GENES & DEVELOPMENT. vol. 17, 2003, pages 2245 - 2258, XP002385472
- PAN S. ET AL.: 'Regulation of Candida glabrata adhesin Epal.' ASM 101ST GENERAL MEETING. SESSION NO. 34/F 21 May 2001, page ABSTRACT F-1, XP009169238
- KAUR R. ET AL.: 'A yeast by any other name: Candida glabrata and its interaction with the host.' CURRENT OPINION IN MICROBIOLOGY vol. 8, 05 July 2005, pages 378 - 384, XP027848292
- CASTANO I. ET AL.: 'Virulencia del hongo patogeno oportunista Candida glabrata' REVISTA LATINOAMERICANA DE MICROBIOLOGIA vol. 48, no. 2, April 2006 - June 2006, pages 66 - 69, XP055103996
- FLINT J ET AL: "Sequence comparison of human and yeast telomeres identifies structurally distinct subtelomeric domains.", HUMAN MOLECULAR GENETICS AUG 1997, vol. 6, no. 8, August 1997 (1997-08), pages 1305-1313, ISSN: 0964-6906

### SEQUENCE LISTING

<110> Instituto Potosino de Investigacion Cientifica y Tecnologica A.C.
<120> "IN VITRO METHOD FOR DETECTING Candida glabrata, DIAGNOSTIC KIT AND USES THEREOF"
<130> P7593EPPC
<140> EP 10753741.7
   <141> 2010-03-17
<150> MX/a/2009/002935
   <151> 2009-03-18
<160> 52
<170> PatentIn version 3.5
<210> 1
   <211> 2225
   <212> DNA
   <213> Candida glabrata
<220>
   <221> EPA1 inter EPA2
   <222> (1)..(2225)
<400> 1
   taaaaccaga aaatataata acttctatag tttagcattg taaaagctgt ggtggctgtt      60
   ttgtttcact gcaatctttc ttgcaggcgg atcacatcac catccgtttt taacagtgta     120
   tattgcctag gtagcatcca tcatcaatac cctttatatc agagctttat acaaataata     180
   ttcctggtca cacttgaaat gagagatatt ataaaaaact tcgggtggta tcataagttt     240
   cagggttttt ggtttacata cgaagcctaa tttgcctttt ctattctccc agagaattca     300
   acttttgagc agggaccatt gttttcgcga ttttcaaaag caagagaaag ttagagcaaa     360
   tcacgaaaat ccagaagata taagaaatct cgaattaaac ttccataata ggtcaattgt     420
   caaaaaaaac tcgaaacaac ttattattct aaacgaatat gatcatatga acataccgtt     480
   ccacgaatat gagagtttat acgactatgg tgcaaggctt aacatgccgc tcatacaggc     540
   acagaagtat ataaacgaag gagaagagaa gataagatat aactaggtag gtaacggact     600
   attgtaatct ttcaggtgaa aagaaaccga gttgggctta ttagaaccag tttatctgcc     660
   acagataaaa ggtataaagg agccctcatt gataatagtc catgaagtta gaccaaagtt     720
   ctagtatctt attctgtata ataaaagagg ataaccagta taatttctat aatataccca     780
   tagaattttg aattaatatc ttatcatcat gtcttctaag tataactgga cacaagaact     840
   aactactcca caaaatcgtt cttagactat cgcaaaacag ggtgatagat actggaacac 900
   cgaatagtag tgatgggcct atcttcactt aaacattagt gtaaagcata ttcaatttta 960
   ttgaccaaaa caataaaagt aattaaaact gtaattaccg aaatttacat gataagtcag 1020
   attattgcac ccgatatgtt tctttcaaga agataatctc atggccgtat agcatatata 1080
   acagtttagg gcagtcccaa ttgtaagcag ttacatagat tgctaacagg aagctttatg 1140
   atctatgtaa aatccagtgt tctaatcgcc tctaaaactt tttttagcta atgttagatt 1200
   aattctaata ctacattcct gataatctgt aaatgatcac aagagagtct tgttgctcga 1260
   acaactgtcc tcgataacat ctcaaagttg aaaaccaaat tctgttggtt aaaaatattg 1320
   tgatattaag tttagactta tcaatgtctt caaaatcata cactaaaata tccaaatgta 1380
   tagaggtagt gtattcgcaa gaaacacggc ctaagacaag taaattttca gaataaattt 1440
   tttagtatag attatagttt tgatatcaag caagatgtta ttggtaatga acataactat 1500
   tataattaag tccaagtaaa agaatctctt ttaatacaat aaattgatca gaatcgtaac 1560
   tggattgata ttcagtaaag cagtatatat caaagtgccc caaccaagat taaatggaat 1620
   accaccccct taacataagg ttccattgca ttcaaaaagg tttccgaaaa atttgtagaa 1680
   tagtgtccaa catattgtta tatctaaaaa atagtttatc atcatataga gggccacatt 1740
   gtttatatag tgcataactt tcgttttttt tagatgtccc atttagggag tctgacttgc 1800
   aacaaaacac tgctcgaaaa tttcgaataa tacaacttaa aatctggtag agtttattga 1860
   attttaggta ctgataaaat ttttataata tgttgaaaaa tggtataact gctcattgtc 1920
   acatccactc aaatgtcgtc aactattaac gaatgcaata taaaaagaca ctagcttagt 1980
   cagtatttca tttcaaattt gttgatactc tggaaagtac aacaaaaaaa aaaaagataa 2040
   tctaattgtc cttgaaaata ttgaacaatc cagaagctaa acatcctaga cattcaaaaa 2100
   caaaaagttt ttagggatat gatctgacat cctttagtta ttttaaaaaa tacaaacagg 2160
   tacatacatt atacatgtat atgacatatc gatcttatgc catcatacat gaaattattg 2220
   agatc 2225
<210> 2
   <211> 2438
   <212> DNA
   <213> Candida glabrata
<220>
   <221> EPAChrH inter TEL
   <222> (1)..(2438)
   <223> Chromosome H(1047924-1050361)
<400> 2
<210> 3
   <211> 8309
   <212> DNA
   <213> Candida glabrata
<220>
   <221> CrK inter EPA22
   <222> (1) .. (8309)
   <223> Chromosome K(5831-14139)
<400> 3
   ttcttgaaaa agcaactatg gaatttgttt ggttccgatg tcaaaaaata aaatactgat 60
   aaggcgataa gttttgacaa tctctcgtta atatttcttt tgtaatgctt cattgataca 120
   tcgactatca agaaaaaaca ttattagtaa attttgaact ttatttaaaa ccatttctga 180
   aatgtatact aagtatttat acttgagaaa aaaaaaacat atctttatct agaaaaaact 240
   gtgtttctgt gatagagtta gaaacattct gaaaattaac tatcaacttt acttgataca 300
   aggggaaaaa ataacacatt tgttgaatat gtttccggaa tttttggtat tacttatttg 360
   ctgcattaca tcatctacat cgctaaaata atactactaa agtaactata tgaaaaacat 420
   ttaatgaaga aattagacaa taaaatatta caattatttt atggtgtttt tgttagcaat 480
   atttgatttt gtgactgagg atttatgggg ttattttaac aaaaaaatga tgtagtctca 540
   aattatttta gtacctgatc aatcttttaa tgagttggta aatataaact atacaaagta 600
   agtacttaaa agaaagacaa gcctctttct caagttcgag tgtgacaata cttattacta 660
   caaagtagac attcgtgtta actaatgttc ttgacttcat tctagccaaa tatgtacata 720
   cacggaactc taaccacgta caaatatatc tttttgcttg tttcatatag tatgattatt 780
   tgctttattt gttagttaac gtgtgaatat aggaatatca tgtttgatgg tagagattag 840
   ttgttcatag cggattggta ttgcattttc cattttctat tttagttatg tgtgtcttga 900
   agcaaataaa tgtatattag aggctatata ttatcacaaa ttcatattgt aattcacaac 960
   tatccaactg cttaattccc aagtttgttt ctttcaatat tcctgatttt cttttgttta 1020
   ctttactgtg atgctatatt agaaaattaa gtcgccaata ttttacctag aataattaat 1080
   ctaaaaagtc atgttaaggt cgtgctctat tactcttgag taatagagcg taatataccc 1140
   acttccggaa ttttctatag aatactggta ataggttgta ttaaagaaat tacaaatttt 1200
   atcggatttt agtagatact tagaagcttt taaactattc aataaaatat tagcatagaa 1260
   gtcattatat atcactcatt ttgtaatcat cttattaatt gagcacataa aaatcagagt 1320
   atttttcttt cttatatgat aatttccatt atagtgattg agatatctag ttttatgcaa 1380
   acatctctta gtagtacaag gttataatag aaatttaatt ttacaaatta tgccacagga 1440
   agaaaaacaa gacaaattta gtatttccta atcaattctt tcataattaa tataatcaaa 1500
   tgatgtcatt tgctagtcat tctcgtaaaa aagttttcag attaagtgtt atttttaatt 1560
   gacagataac tagtacgata acaaaacaaa aatggagata aaagcacctt tcgcaaaata 1620
   cagttttggc tacaaaatat atctaacatt tagttatctg aatttcttag atacatatct 1680
   gaattaaata aataaagaca cactaatgtc cggaacaagc aaatactatc ttgtgtttat 1740
   tcaagccaaa aagggataaa aatcattact atctggagaa ttagcttggg tagcagtatt 1800
   tattcatagt tagatacaac gaagtgtgtc aaaaaaacaa tagctgaaat actggcttct 1860
   cgcttaaatt ctgttcatac attgatttta gtgtaccact aagacattat actagataaa 1920
   aatgtaaagc aaatggtaga atatatcaaa aatgatggtc ataaaataca ttggcatttg 1980
   gagatggtca gataataatg caaatatgaa gaattaacaa agtacctagc atatgttatt 2040
   gaaacaatcc ttatcactgg tgctttattt tgtcattcat gaaatcgaaa tagagcttat 2100
   attatatctt catttatgaa gcaaatatta cttcattgtg ttccttgaga atataataga 2160
   atcaccagca agcaatgaaa ttttgaacta catttttagc gtcacgcggc aacaataatt 2220
   tatggatatc aatatcacga aataggatta atagaaaaca taatgaggaa aatattggtt 2280
   accaatatta tttacatatg agtaacctct aacctccttc tccaagaaca ctagtagatt 2340
   taattacaat tttacaacta cttttcccta ctttacacat cgttgaagac atcgttccaa 2400
   aaaaaaatgt aatgtaattt tagtaaaaga atgatattca taaatttcga ctgtgtgctt 2460
   atcacacata tcgctgataa atgatttgtt aactgttttg agaagcaaaa atatattgtc 2520
   atgcattttc atggcttttt gacatcttat ttcattataa attacattca aagatctaga 2580
   ccccaattaa gattcaagct aaatcgtaac tactttaaac acactcaaca aaccttaaca 2640
   tgatgaagac agatatttaa cgaccaattt taagtagtat attgtttgag aagattgcga 2700
   taactaatct tttagacaag aataagttag tttactattg gtttgcccag catttacaat 2760
   ggcattgggt aatattaata gtatttcatt accaagtctt cccttcatca gcaatattca 2820
   atcgtagctt ccttattggt tcactgttaa agtaaattca acatttcatt tatttcgtga 2880
   cgtcattgta gtgccgtacg aagaattagc aattttttgt tcgtgacatg ttttgtaatt 2940
   gtttcctatt ttggctagat tcatgaaact ttttgtcatc aaatattttt ttgtaaattc 3000
   aaaatagttt cttcaaaaaa atttggctgg catatttact ttttatgacg tcacttcttc 3060
   tccaagaaat aaatgttagg gtagcaccta ctaggtattg tggattatat tttatcaatc 3120
   caatcacagt tgttttcaga tattggaaaa ttgtgattct aaagagctgt aacataaata 3180
   tttctactta ttctacaagt tgtttcaacc tctgctgtta ttacaagtaa taaccaataa 3240
   gttattttta ctcgacctac tttggttaaa aacattatta ttccggtata ttgttcaaat 3300
   agatgcaaac taatcgagat cataaaagtt tgctttttac tgattgaata aattctttca 3360
   tttataatac ctttttgtat tttggtatag ctattattta tgtaattgga tgtttccatc 3420
   aacgaaattc aataatttta taaggcagta ctaagcgttc gttagagaaa tatgaaaata 3480
   gatatgtcat aaaaatgcga tctcaaaact ttttgttaaa cactgttgat actcatatta 3540
   gatttgtagt aacaggagct gatgttattc atgattttgc taagttatac taattagtag 3600
   taaagtctag atattataac tttcaacatg gtttagttat tacatatata tctattaaaa 3660
   atatcattga aggagccgta attgagttgt tgttcagttt taattcattt gcagtatttg 3720
   tgtttttttt agcaataaac ttctgggcaa agaggaaaca taattgccgt tttttcatag 3780
   tcatattgtt ctgagatgca atgtcgaaat acatgtattc tggtaattat ttatcattcg 3840
   ttctctagat tttttgtatg ctttcgtaca aattatcgcg ggtaaagagt aagatatgaa 3900
   cgtgattctc gttgaaacta atatatggat ttttgttgag atagtctaag acgatcttgt 3960
   ggagtagtta gttcttgtgt ccagatacat agaagacatg atgataagat attaattcaa 4020
   gactctaagg gtatattata gaaattatac tggttatcct ctttcattat acagaataag 4080
   acactagaac tttggtctaa cttcatggac tattatcaat gaggactcct tccttttatc 4140
   tttgacagat aaattggttc taagaagccc taactcggtt tcttttctga ttagtaatct 4200
   ttcaggtgat tctttttact tgaaagtgac catagtccgt taccggccta attctatctt 4260
   atcttatctt ctccttcgtt tatatacttc tgtgcctgta tgagcggtac tataatgcct 4320
   ggtgtcattg tcgtttaaga cgttcgtatt catggcacgg tatgatcata tgatcattgt 4380
   cgtttagaat aataagtagt ttcgagttta tctcaacaac tttttatcaa ttatttaccc 4440
   ggctctatat tggatgagta tttgtcatca taacctgttg gaacatccgc tttcttagct 4500
   ttcaacagta aattcggcta atctgcaata aatcagcttt gataaatagt gacgactgat 4560
   aaaaatatat gatacaaata tagaattgta ttatgcaata taattcaagt tccataagta 4620
   aatataacat atatgtggta aaatgattat ggtaagatta ctcaaatctg gagttttatt 4680
   tattatttta tatgtattgt ttaaagattc cttatcattt agtgtttcat ttaccctatc 4740
   aaaaatgata actacatatg atcgattagt tatgccttat tttatatgtg tctctgtagt 4800
   actgtgcata agtctataaa gtgcctatta ttggttattg tcaatgatta ctggttgctc 4860
   atagagatcg cacatgttac agatggcatt tgcacgtgat tatgctatgt ccgtctagaa 4920
   taatgattaa tactagtata tcaaacatat tctgtaacgg ccaaatttgg taaaatttga 4980
   ttgacttaac aagacacatt gcttcttttt aatgggggtg ggagaaaaag gtacaaatgg 5040
   gtttaaaaag tacctgttct attttatttc agtagaaata ctttttatat tttcatctta 5100
   caagttggta atgttttagt atattggtta ttgcctaccc cacggtttgc aattcaattg 5160
   gtaacagtag gttaattaag agacaagaaa attaataaat aataatacta tttttgttat 5220
   atatgaaatc cacaaataat agattgcata ttgttcaatg gagtggattt tattatcatg 5280
   aaatgaaaca ttacttagta atttagtagt ttccttttgt aatttcatta ttgttgggtt 5340
   ctgattaata caaatcgtat tacatttcta ttcctgtctg ttcttgagag gtcttttagc 5400
   attagttagg tgttctctgt atgcgaataa aattgtggag gcaatttatt gtgtttatta 5460
   acgttttcaa attaatttgt gatcataaat ttctggccaa gtattgcttc ttaattaatt 5520
   atttataaat tatttatcaa gtaactttac taaacactcc ttatctttct ggatttttct 5580
   ttgattaaac tacgggttaa tataattaat catagttgat agtttcccac cttttattat 5640
   tgttattata caaatcatat atatgactag atggtaaaat ttgttgttag taaggatgct 5700
   acatagaatg atatccatcc ttcgtaaaca ctctgcctag agttaaaaag tgagtatttt 5760
   tatggaaatt agcatttgat aattatctaa ttggtttttc aactactatt tgatagcata 5820
   ttaatttcta gttggtattt ggctaaaagt ttctcaatat ttgaaaacag agccttagca 5880
   caataaatta ctaaattatt tgactatgag attgttcatt tttattttaa attttggtag 5940
   ttatttaaaa gtcttaattt tatgtctgct cgggttaatt agcatgattg attataatca 6000
   gtatctagct cttagacaaa cacaagcagt tatcggttga aaattaaagg atttacaaag 6060
   tcatattatg tcgtggagca gaagaataaa gtactattga atacagtaca atcttagtag 6120
   tggtcagagt gcgtagtaac ttatgcacgt aatatgacgt ataaaaaatt catatcaaac 6180
   ctatgataat gttccgtgta agataatgaa gaataatgga gttttttcag aaccccgtca 6240
   taaaatcata tcgtggaagg attgctgtgt gaacctgttc aactgacgga aaatccatag 6300
   gatgcgggtt gaactgtaat gatgctggcc actaaaaact gtaaagcaag tatatacatg 6360
   cattacgagt ctagactgtt ctttttctag ttgatgatgt cccttataag ccaagatacc 6420
   atatcaatat ttattatctt gtatcttctt ggctcgcatt tgtaatacat catttctttc 6480
   ttcaggattt tgctttcttg agaaaatgat tttcaaatag aaaaaagatt ttcataaaga 6540
   aaagggtcaa aacggatata agagttagat atcgaggctg tgcagccact tgagaggagt 6600
   gatctatcag aaaaataatt tccgtacggc attaactatg cgttgataat gataaaaata 6660
   ggggttcatc ccttaaatgc aaggggtttc ctttgctatc tgagaaccct tgtcaattgg 6720
   ggtattacgt atgatgttaa ggccaggaga ctattatatc agcttctctt tctacagtaa 6780
   gcacatgggt tccagctacc acaagttgca attgcaataa gaaaagagtg gttgacaggc 6840
   cagaaaagta aagaaataca cggaaatgac atatcccctc ccagtattat ggatgtggca 6900
   atgacgagat tatgttcgtc caatcaaggg tttaatatta gtcttgatca tactaagact 6960
   aatatttggg gtaaagggat tgttcattta ttcatgtatt atgcaggatt tgcattgatt 7020
   gcctgagaga caggcaagag ttaaggagtg agatggctgg tagaatcttc gtgctttcct 7080
   tcgtgtgttt tattgatgtt tcttgtgtga ttcggtaaac tccttcgact ggactatatt 7140
   gatcgtcatg agtcttacga taattataca agctgatagt tgtgtttgaa gtaatgagtt 7200
   atccagtata cttattagtc tgtggaagga cgtagttatt gcaagcacag ttggtcactg 7260
   ttttagccat tttaagggtg tctttcgact atcccaaaga catcggggaa gaaattaaga 7320
   gacatatact agtcgtcata gtactatatt tgccttattt ggcaaagtga ttgtatttga 7380
   tgttaccgaa agcgatacag accactatta gatgttcctg caaatccttg ctccctactc 7440
   taatgcagaa attttcagtt aagctgtctt ttgcaattct gagtagactc tactacatga 7500
   tatgaaattt acaatttatc aaacaaatgt aaatacagtt acgaaggatt taccttgcaa 7560
   aatcagaaac tgaagttttt ttttgttatg ctatatgtga tatcagaaca gcgtttgacg 7620
   gtgacatcat aattattgcc agagtattac aagatcttaa ttaataccta gtagaattaa 7680
   tatcaaatac atttaactcc aaagagaaat tgtaacttgg ttaatgatga ttttccataa 7740
   ctaatcttct gaaattattc ctgggcctta tctaaattga agatattgat taagttgagc 7800
   ttaggatatg atactatggt aatactgttg ttaatgattg attatcaaag tattgtcatg 7860
   aagacttcct taaagacttt cttaaagact ttcttaaaga ctataattta gattttaata 7920
   aagactttca tgaagatttt aataaagact ttcgtgaaga ttttcatgaa gattttccat 7980
   aagatttttt aaaatgttat tactgttaag tcttgtgaaa agcgtcaaca gaattcgatt 8040
   tagatctttc taattttcaa gagcataggt gaagttttaa ccgaggtaaa tccccaataa 8100
   ttttttacat ttcatgacta agagtagttt ttatcaagtt atcattctta ctgatacaaa 8160
   attgaacata taaaaggatg aacaatattg aagtttttga taaaataagt attccaattt 8220
   cttataatca taagatagcc catcacaaaa gacaactatt gaaagtaacc agattgaaaa 8280
   tcacatatta tcaatattga atatcggaa 8309
<210> 4
   <211> 2432
   <212> DNA
   <213> Candida glabrata
<220>
   <221> EPA6 to Tel
   <222> (1) .. (2432)
   <223> GenBank Accession No. AY646925 (15567\20513136), homologous to the intergenic region between EPA6 and the chromosome C telomere published in Genolevures
<400> 4
<210> 5
   <211> 2498
   <212> DNA
   <213> Candida glabrata
<220>
   <2211> EPA7 to Tel
   <222> (1) .. (2498)
   <223> GenBank Accession No. AY646926 (6089-8586), homologous to the intergenic region between EPA7 and the chromosome C telomere published in Génolevures
<400> 5
   aaatatgtga taccacattt cctaagatag ggttgtgaaa tattatcata cataagattt      60
   cttcatacag acctaattca aatgtgggct gagtgagttt aagtttttat catttttact     120
   gatacatgcc ttaacgatta tcacacatta aaaaaatgaa tgaatactaa gcatagctat     180
   cttattcgtt attgagaaaa agtggttttg aaatatactg ggtctaacat atgccaagaa     240
   tattgtggat atattgtatt taaatttatt tgtcagtgaa tttattgata aacataacat     300
   agtggtatca attcttttga aaagctacat atttctacct tatttttttt tccattctca     360
   tttattgaaa gaatttattg tcccttaaac tgttgagctt gaatctacga aagcaagtag     420
   atgatatatt ccacgtgata tttatattat ttacgttcta tacatatatt gataggataa     480
   tgaatatctt tcgtacagaa agagttgaat ttgcaggaat atagtactgt gaagtaagta     540
   ccagattttg gtactagttg tacggaaagg actgttacca aaacattatt atcagtgaca     600
   atgaagattt gggttgtatt ttattgagaa gatttccaat agcagattca ctaaaacgaa     660
   aatacatgtg atgattttgt gccacaaatg tgaatgtccg tttgctgcga taatctaaga     720
   cgatcttgtg gagtagttgg tacttgtgtc cagttatact tggaaaacat gatgctaaga     780
   tattaattca aaattctaat ggtatattat acaaattata ctgtttattt ttcttttact     840
   atacaaaata agatactaga attaaggttt agctttatag gctaatggag atcagggttc     900
   cttttatacc tttcatcttt tggcagataa actggttcta agaagcccta actcggtttc     960
   ttttctgatt actaatcttt cagataaaaa ttcatttcac ctgtaagatg ataatagtcc    1020
   gtcacaggcc tagttatatc ttatctaagc ttcgcttctc tttcgtttat atacttctat    1080
   gtctggatga gcggtattat aatgcctggc gtaattggcg tttacgacgc tcttcttcgt    1140
   ggcacggtat cagcatatga tcatattcgt ataaatcaat aagtagtttc gagttaatct    1200
   aagcatctac taccgtaaca cttcacatta tgatatttgt tgaaatacgt tgaaataaat    1260
   tcgaagcatc tactaccgta acacttcaca ttatgatatt tgttgaaata cgttgaaata    1320
   aattcgaagc atcttattat tctaaacgaa gatagtcata tgaatatatt gtggcacgaa    1380
   tatgagtggt atactataat gattaaaggc ttaatagtac cgctgataca ggcacagtaa    1440
   taaattcaca aagtcatcga taataagatt aaaatggaca ggtaacagac taaggtcact    1500
   ttccaggtga agagaaagcg agttaggact tctaaaaaac agtatatctg ccaaaaataa    1560
   atggtataaa gagccgttat acatcatagt ctatcatgtt aaaggtaagt tctcatatca    1620
   ttttatgcat ttcataacta tagttgcaac aatatcttat cagttagtct tacttacaga 1680
   cttcgacgcc agaaatgaca gatcactacc aacgtctata tcattttaac accacatgtt 1740
   aatatttata attctaaacg taagtctaac ataaggctag aatttcacaa attgttggct 1800
   tgaaattgag caaaattgaa aagtgacgaa tacaaaattc ctaaccaaaa tattaagttt 1860
   ttatataaca ataagtcgat tatgaccact agtcagtaca aatatcaaca ctattatttt 1920
   acagaattta ttttaattta ccataccaaa taaggagaga tattctgatt tgttgtttat 1980
   ttcttggtaa atatggagaa taaataaata ttgttcttcc ttcatattct taggatatag 2040
   aagtatgata acatcaatac aaaaattaat tcaattaaag tataaaaaat ttcatatatt 2100
   aaaacctaat atgatacaaa taataaaaat cggaatgatg aaagaaagga aaaacagtat 2160
   tgaacttggt aaagaatata ataattcaga aggaaaccac attcatcatg cgaatagcca 2220
   ttgaaaaaag gttcaagtga taagttacac aaaaactatc aatacttata acacagagat 2280
   gcataaatat tttatacaag aaaagactat taccagatta taatcgaaaa aaataaagta 2340
   aaaatatatt acacaaaata ctaatgacgt cttatcaata aatataaggg accgttaaaa 2400
   agaaatagaa tttaacatta ttgtaagaaa gtaaaaattg aattaatgta tttacattat 2460
   gataatcaat tttatatatt ttctagtgct ctaacttt 2498
<210> 6
   <211> 20
   <212> DNA
   <213> Candida glabrata
<220>
   <221> oligonucleotide
   <222> (1) .. (20)
<400> 6
   cgctcataca ggcacagaag 20
<210> 7
   <211> 30
   <212> DNA
   <213> Candida glabrata
<220>
   <221> Oligonucleotide
   <222> (1) .. (30)
<400> 7
   ccaaagttct agtatcttat tctgtataat 30
12/230> 8
<211> 26
   <212> DNA
   <213> Candida glabrata
<220>
   <221> Oligonucleotide
   <222> (1) .. (26)
<400> 8
   gtggagtagt tagttcttgt gtccag 26
<210> 9
   <211> 22
   <212> DNA
   <213> Candida glabrata
<220>
   <221> Oligonucleotide
   <222> (1) .. (22)
<400> 9
   ataccactca tattcgtgcc ac 22
<210> 10
   <211> 24
   <212> DNA
   <213> Candida glabrata
<220>
   <221> Oligonucleotide
   <222> (1) .. (24)
<400> 10
   gtggagtagt tggtacttgt gtcc 24
<210> 11
   <211> 27
   <212> DNA
   <213> Candida glabrata
<220>
   <221> Oligonucleotide
   <222> (1) .. (27)
<400> 11
   acagtgtata ttgcctaggt agcatcc 27
<210> 12
   <211> 23
   <212> DNA
   <213> Candida glabrata
<220>
   <221> Oligonucleotide
   <222> (1) .. (23)
<400> 12
   cgggtggtat cataagtttc agg 23
<210> 13
   <211> 21
   <212> DNA
   <213> Candida glabrata
<220>
   <221> Oligonucleotide
   <222> (1) .. (21)
<400> 13
   taagtcagat tattgcaccc g 21
<210> 14
   <211> 21
   <212> DNA
   <213> Candida glabrata
<220>
   <221> Oligonucleotide
   <222> (1) .. (21)
<400> 14
   aaatccagtg ttctaatcgc c 21
<210> 15
   <211> 27
   <212> DNA
   <213> Candida glabrata
<220>
   <221> Oligonucleotide
   <222> (1) .. (27)
<400> 15
   ccaaatgtat agaggtagtg tattcgc 27
<210> 16
   <211> 24
   <212> DNA
   <213> Candida glabrata
<220>
   <221> Oligonucleotide
   <222> (1) .. (24)
<400> 16
   ccaaccaaga ttaaatggaa tacc 24
<210> 17
   <211> 20
   <212> DNA
   <213> Candida glabrata
<220>
   <221> Oligonucleotide
   <222> (1) .. (20)
<400> 17
   tcacatccac tcaaatgtcg 20
<210> 18
   <211> 22
   <212> DNA
   <213> Candida glabrata
<220>
   <221> Oligonucleotide
   <222> (1) .. (22)
<400> 18
   ctcaaaagtt gaattctctg gg 22
<210> 19
   <211> 22
   <212> DNA
   <213> Candida glabrata
<220>
   <221> Oligonucleotide
   <222> (1) .. (22)
<400> 19
   gttaagcctt gcaccatagt cg 22
<210> 20
   <211> 23
   <212> DNA
   <213> Candida glabrata
<220>
   <221> Oligonucleotide
   <222> (1) .. (23)
<400> 20
   atgttatcga ggacagttgt tcg 23
<210> 21
   <211> 22
   <212> DNA
   <213> Candida glabrata
<220>
   <221> Oligonucleotide
   <222> (1) .. (22)
<400> 21
   gcaagtcaga ctccctaaat gg 22
<210> 22
   <211> 21
   <212> DNA
   <213> Candida glabrata
<220>
   <221> Oligonucleotide
   <222> (1) .. (21)
<400> 22
   tgagtggatg tgacaatgag c 21
<210> 23
   <211> 20
   <212> DNA
   <213> Candida glabrata
<220>
   <221> Oligonucleotide
   <222> (1) .. (20)
<400> 23
   gacctaattc aaatgtgggc 20
<210> 24
   <211> 24
   <212> DNA
   <213> Candida glabrata
<220>
   <221> Oligonucleotide
   <222> (1) .. (24)
<400> 24
   taacatatgc caagaatatt gtgg 24
<210> 25
   <211> 21
   <212> DNA
   <213> Candida glabrata
<220>
   <221> Oligonucleotide
   <222> (1) .. (21)
<400> 25
   tcaagctcaa cagtttaagg g 21
<210> 26
   <211> 21
   <212> DNA
   <213> Candida glabrata
<220>
   <221> Oligonucleotide
   <222> (1) .. (21)
<400> 26
   tgtacggaaa ggactgttac c 21
<210> 27
   <211> 19
   <212> DNA
   <213> Candida glabrata
<220>
   <221> Oligonucleotide
   <222> (1) .. (19)
<400> 27
   accctgatct ccattagcc 19
<210> 28
   <211> 25
   <212> DNA
   <213> Candida glabrata
<220>
   <221> Oligonucleotide
   <222> (1) .. (25)
<400> 28
   tcacctgtaa gatgataata gtccg 25
<210> 29
   <211> 19
   <212> DNA
   <213> Candida glabrata
<220>
   <221> Oligonucleotide
   <222> (1) .. (19)
<400> 29
   catatgctga taccgtgcc 19
<210> 30
   <211> 24
   <212> DNA
   <213> Candida glabrata
<220>
   <221> Oligonucleotide
   <222> (1) .. (24)
<400> 30
   ggtatcagca tatgatcata ttcg 24
<210> 31
   <211> 24
   <212> DNA
   <213> Candida glabrata
<220>
   <221> Oligonucleotide
   <222> (1) .. (24)
<400> 31
   cttaatagta ccgctgatac aggc 24
<210> 32
   <211> 22
   <212> DNA
   <213> Candida glabrata
<220>
   <221> Oligonucleotide
   <222> (1) .. (22)
<400> 32
   cttacttaca gacttcgacg cc 22
<210> 33
   <211> 20
   <212> DNA
   <213> Candida glabrata
<220>
   <221> Oligonucleotide
   <222> (1) .. (20)
<400> 33
   tcctttcttt catcattccg 20
<210> 34
   <211> 23
   <212> DNA
   <213> Candida glabrata
<220>
   <221> Oligonucleotide
   <222> (1)..(23)
<400> 34
   ttctatttct ttttaacggt ccc 23
<210> 35
   <211> 32
   <212> DNA
   <213> Candida glabrata
<220>
   <221> Oligonucleotide
   <222> (1) .. (32)
<400> 35
   taataacttc tatagtttag cattgtaaaa gc 32
<210> 36
   <211> 26
   <212> DNA
   <213> Candida glabrata
<220>
   <221> Oligonucleotide
   <222> (1) .. (26)
<400> 36
   gatctcaata atttcatgta tgatgg 26
<210> 37
   <211> 21
   <212> DNA
   <213> Candida glabrata
<220>
   <221> Oligonucleotide
   <222> (1) .. (21)
<400> 37
   aaataaaata ctgataaggc g 21
0> 38
<211> 18
   <212> DNA
   <213> Candida glabrata
<220>
   <221> Oligonucleotide
   <222> (1) .. (18)
<400> 38
   tcgaacttga gaaagagg 18
<210> 39
   <211> 24
   <212> DNA
   <213> Candida glabrata
<220>
   <221> Oligonucleotide
   <222> (1) .. (24)
<400> 39
   caaacatctc ttagtagtac aagg 24
<210> 40
   <211> 25
   <212> DNA
   <213> Candida glabrata
<220>
   <221> Oligonucleotide
   <222> (1) .. (25)
<400> 40
   taaatgttag atatattttg tagcc 25
<210> 41
   <211> 18
   <212> DNA
   <213> Candida glabrata
<220>
   <221> Oligonucleotide
   <222> (1)..(18)
<400> 41
   cctctaacct ccttctcc 18
<210> 42
   <211> 21
   <212> DNA
   <213> Candida glabrata
<220>
   <221> Oligonucleotide
   <222> (1) .. (21)
<400> 42
   tctttagaat cacaattttc c 21
<210> 43
   <211> 19
   <212> DNA
   <213> Candida glabrata
<220>
   <221> Oligonucleotide
   <222> (1) .. (19)
<400> 43
   aaatatcatt gaaggagcc 19
<210> 44
   <211> 25
   <212> DNA
   <213> Candida glabrata
<220>
   <221> Oligonucleotide
   <222> (1) .. (25)
<400> 44
   ttgtatcata tatttttatc agtcg 25
<210> 45
   <211> 23
   <212> DNA
   <213> Candida glabrata
<220>
   <221> Oligonucleotide
   <222> (1) .. (23)
<400> 45
   catactaaga ctaatatttg ggg 23
<210> 46
   <211> 20
   <212> DNA
   <213> Candida glabrata
<220>
   <221> Oligonucleotide
   2> (1) .. (20)
<400> 46
   caataattat gatgtcaccg 20
<210> 47
   <211> 18
   <212> DNA
   <213> Candida glabrata
<220>
   <221> Oligonucleotide
   <222> (1) .. (18)
<400> 47
   ttgtcttttg tgatgggc 18
<210> 48
   <211> 23
   <212> DNA
   <213> Candida glabrata
<220>
   <221> Oligonucleotide
   <222> (1) .. (23)
<400> 48
   caacatgtac agtaatacaa acg 23
<210> 49
   <211> 18
   <212> DNA
   <213> Candida glabrata
<220>
   <221> Oligonucleotide
   <222> (1) .. (18)
<400> 49
   ctgggtaact tcgattgg 18
<210> 50
   <211> 20
   <212> DNA
   <213> Candida glabrata
<220>
   <221> Oligonucleotide
   <222> (1) .. (20)
<400> 50
   agacatgcta ctgtgattcc 20
<210> 51
   <211> 20
   <212> DNA
   <213> Candida glabrata
<220>
   <221> Oligonucleotide
   <222> (1) .. (20)
<400> 51
   tgggagttag acatacatgg 20
<210> 52
   <211> 20
   <212> DNA
   <213> Candida glabrata
<220>
   <221> Oligonucleotide
   <222> (1) .. (20)
<400> 52
   atactgcaca gatattgtcg 20

## Description

### FIELD OF THE INVENTION

The present invention relates to biotechnology, especially molecular diagnostic methods in vitro.

### BACKGROUND

Historically, *Candida glabrata* has been considered as a relatively non-pathogenic fungus in the normal flora of healthy individuals. That is, they rarely cause serious infections in humans. However, under the increased use of immunosuppressive therapy with antifungal therapy with broad-spectrum, frequency of infections caused by mucosal or systemic routes by *C. glabrata* has increased significantly. In fact, depending on the site of infection, *C. glabrata* is usually the second or third most common cause of infection after *C. albicans,* representing 17 to 23% of cases of candidiasis in the bloodstream in patients aged 16 to 65 years (MA Pfaller, et. al, Journal of Clinical Microbiology, Mar. 2002, p. 852-856 VOI. 40, No. 3). In addition, infections with *C. glabrata* are common in abnormal hosts, eg, immunocompromised persons, patients with diabetes mellitus, cancer patients or, on the other hand, in neonates or elderly people. It is also common in hospital settings and in patients who have undergone surgery, have been handled by hospital staff (catheterization) or been hospitalized for prolonged periods and mortality in such patients is equal to the rate of infections caused by *C. albicans.*

Moreover, in the field of veterinary medicine it has been reported yeast infections in cows, where a significant percentage corresponds to *C. glabrata.* (Costa, GM et al. Ciena. Rural, 2008, Vol. 38, No. 7, pp. 1938-1942). It is considered that a rapid and accurate diagnosis in mammals is necessary to bring down the incidence of *C. glabrata.*

Infections of *C. glabrata* are difficult to treat and are generally resistant to many antifungal agents on the type of azoles, especially fluconazole, which is the most prescribed antifungal for candidiasis cases. Therefore, to be resistant to common antifungal and lack of a rapid and accurate diagnosis, infection of *C. glabrata* have a high mortality in hospital patients.

### Diagnostic methods for C. glabrata

Three groups of diagnostic methods for candidiasis are reported in the literature by selection in culture medium, biochemical tests and molecular tests.

Among the methods for selecting the culture medium are reported, by way of example, Mycosis IC / F, the Plus Aerobic / F standard and Chrome-agar, among others. The main feature of these culture media is that differentiate various *Candida* species by colony type and color. However, incubation times ranging from 24 to 43 hours, or up to 7 days and also the sensitivity and specificity do not reach optimum percentages (ranging from 37.5% to a maximum of 96%). (J. Clin. Microbiol. Feb. 2004, pp 773-777, Vol. 42 No. 2, J. Clin. Microbiol., Jan. 2004, pp 115-118, Vol. 42, No. 1; J. Clin. Microbiol. Oct. 2003, pp. 4714-4717, Vol. 41, No. 10). Therefore, even when they are economic methods for diagnosis, the results are not specific and very time consuming, so in the case of *C. glabrata* involve compromising the patient's life.

In terms of biochemical methods, the following are included: the systems API 20 or API 20C (Bio-Merieux Vitex Inc.) are based on carbohydrate assimilation tests for identification of clinically relevant yeasts, the incubation time is approximately 72 hours, but is always recommended to correlate with morphology in cultures or similar methods, so it is not 100% specific for identification of fungi. There are improved tests such as Vitex Yeast Biochemical Card, which is an automated system wherein 26 conventional biochemical tests and 4 negative controls are done, with an accuracy greater than or equal to 85%, but if accuracy is less, additional morphological testing should be made (J. Clin. Microbiol, May 1994, pp. 1184-1187, Vol. 32, No. 5; J. Clin. Microbiol., June 1999, pp. 1967-1970, Vol. 37 No. 6; J. Clin. Microbiol., April, 1998, pp. 883-886, Vol. 36 No. 4; J. Clin. Microbiol. May 1998, pp. 1443-1445, Vol. 36, No. 5).

Other methods are based on the uptake of trehalose in *C. glabrata,* and lack of assimilation of sucrose. However, in these cases there are false positives with *Candida tropicalis,* a large amount of inoculum is needed and the results often depend on the culture medium. In some cases, the inoculum density makes difficult to read colors in the plates and the laboratory worker must be very experienced to standardize the technique (J. Clin. Microbiol., Mar 2001, pp. 1172-1174, Vol. 39, No. 3). Among these methods are the Rosco Diagnostic Plates, Glabrata RTT and Dipstick Test, etc.

From the previous reports it is derived that even if biochemical tests are relatively quick, additional tests are always recommended which increase the time to obtain results, they depend on the skill of the laboratory worker and / or the type of medium, so a system that identifies *C*. *glabrata* is required without additional testing, improving efficiency and to provide clinically relevant information quickly and cheaply.

Among the molecular methods, the literature describes (J. Clin. Microbiol., December 2005, pp. 5912-5915 Vol. 43 No. 12) a method to identify 7 *Candida* species by denaturing HPLC, which allows high-resolution analysis of PCR products from blood cultures, where the amplifications are ITS2 variable regions with a subsequent analysis with the WAVE microbial analysis system. Denaturing HPLC separates PCR products which are stained with interspersed staining and visualized with a fluorescence detector. This method is expensive and requires a day's work.

Moreover, it has also been reported using fluorescence in situ hybridization (FISH) with PNA probes for *C. albicans* (J. Clin. Microbiol. June 2002, pp. 2182-2186, Vol. 40, No. 6; J. Clin. Microbiol., Nov. 2007, pp. 3802 - 3803, Vol. 45, No. 11; J. Clin. Microbiol., June 2005, pp. 2909-2912, Vol. 43 No. 6; J. Clin. Microbiol., Sep 2006, 5 pp. 3381-3383 Vol. 44, No. 9), where variable data are reported in terms of specificity and sensitivity of the method, since in some reports there are inconsistencies with isolates that were positive, but when biochemical testing are performed, species other than *Candida* were detected, in some cases different *Candida* species were not detected in mixtures, and the reliability of the test depends on the design of oligonucleotide probes. In one of these reports the need for specific probes for *C. glabrata* is highlighted.

Other examples of molecular methods for detection of *Candida glabrata* based on the identification of the inner regions of the transcript spacer (internal transcribed spacer regions ITS) of ribosomal DNA as those reported in U.S. Patent 5,631,132, U.S. 5,688,644, U.S. 5,426,027 , U.S. 6,858,387 (equivalent to WO 00/73499 A2), U.S. 7,427,472, and WO 98/11257. Also, there have been methods that detect a hypervariable region within the region ribosomal 28S as reported in U.S. Patent 5,707,802, U.S. 5,763,169 and U.S. 6,180,339. However, the reliability of diagnostic methods through the detection of ribosomal DNA regions is lower in a mixture of phylogenetically close organisms.

Moreover, there have been methods of identification of *Candida glabrata* by detecting regions of certain proteins. U.S. Patent 6,497,880 reports a method by detecting gene sequences that code for proteins in response to heat (heat shock proteins, Hsps). On the other hand, U.S. Patent, 6,017,699 reports a method for identifying candidas by detecting gene sequences of chitin synthase 1, CHS1 (chitin synthase). Methods for identifying candida by specific sequences in conserved genes can reduce their reliance on a mixture of phylogenetically close organisms.

### Adhesins in C. glabrata

The lectin-like adhesins are proteins that have been described in several species of pathogens, including *C*. *glabrata* and *C. albicans.* These species adhere to host epithelial cells through the recognition of cell wall carbohydrates therein.

In C. *glabrata* a cell wall protein called Epa1 was identified which is the primary adhesin responsible for adherence presenting cells of *C. glabrata* to epithelial cells in vitro. Epa1 is a lectin that recognizes glycoconjugate of N-acetyl-lactosamine present on the surface of mammalian epithelial cells (Cormack et al., 1999).

EPA1 gene was initially identified by searching for mutants of *C. glabrata* unable to adhere to epithelial cells in vitro, and showed that a deletion of the EPA1 gene reduced the adherence of *C. glabrata* to undetectable levels. Also, EPA7 and EPA6 are genes encoding other adhesins (conferred adherence to *cerevisiae* when expressed ectopically), and are located in subtelomeric regions (Gene Bank sequences AY646926 and AY646925, respectively). This location has meant that EPA6 and EPA7 (and other subtelomeric adhesins) are not expressed in vitro. For urinary tract infections, however, is induced at least Epa6 (Domergue et al., 2005).

The genome of *C. glabrata* contains a family of cell wall proteins of at least 23 members. EPA1 is grouped with two other related genes, EPA2 and EPA3, in a region adjacent to the right side of the telomere on chromosome E (Gene Bank sequence AY344226). At the right side of subtelomeric region of chromosome I, we also found genes EPA4 and EPA5 (De Las Peñas et al., 2003) (Gene Bank sequence AY344225). In total, from the 23 members of the family Epa, at least 17 are located in regions adjacent to telomeres, and during in vitro growth are transcriptionally inactive (Brown et al. 2005; De Las Peñas et al., 2003). This negative regulation depends on the repressive structure of chromatin in regions near telomeres (subtelomeric silencing).

By bioinformatic analysis, it has been deduced that EPA1, EPA6 and EPA7 are more homologous to each other at the amino terminal end.

In order to overcome the technical challenges of specific and sensitive identification of *C. glabrata,* the present invention arises from a strategy analysis of the intergenic regions of the adhesins of *C. glabrata,* especially the variants of the regions containing the sequences of the negative elements (EN) to identify *Candida glabrata* and from the evidence of its usefulness in the identification of this organism It is worth mentioning that this strategy has not been addressed in any report of prior art, so it is novel, not obvious and subject to industrial application.

### BRIEF DESCRIPTION OF THE FIGURES

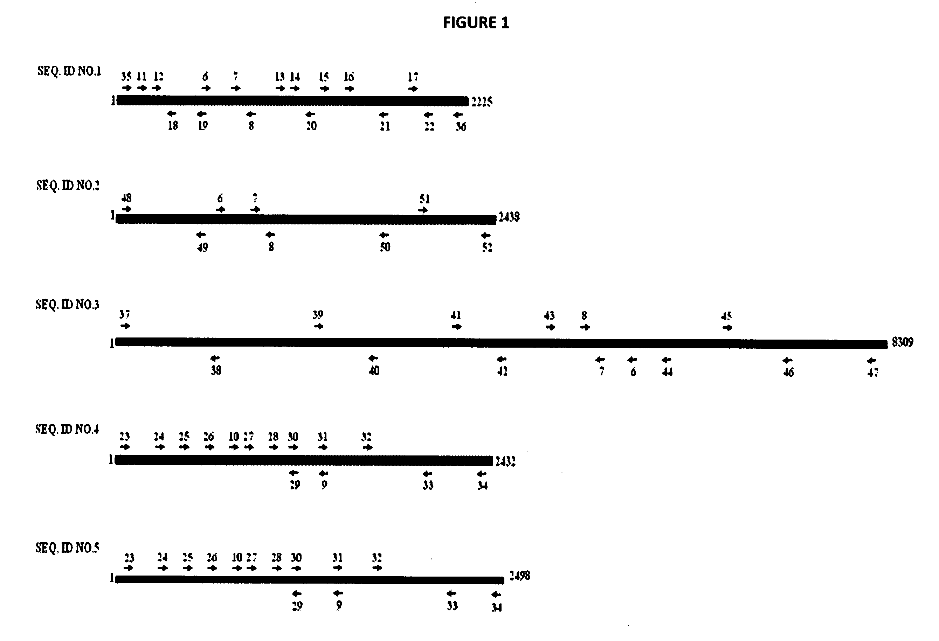

Figure 1. Diagram showing the location of oligonucleotides generated along the SEQ. ID. Nos. 1 to 5. The numbers correspond to SEQ. ID. Nos. 6 to 52 and their position and orientation of the sequences SEQ. ID. Nos. 1 to 5.

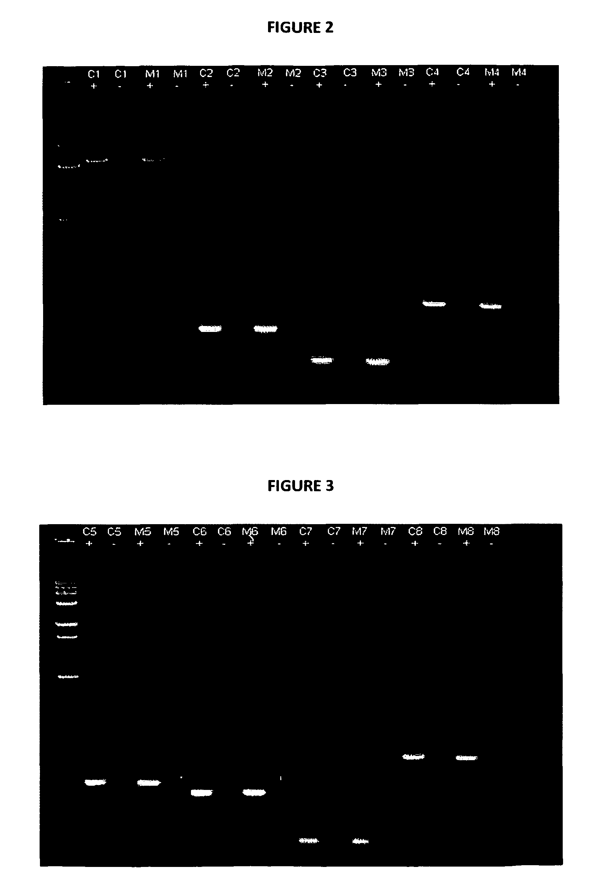

Figure 2. 2% agarose gel, showing different pairs of oligonucleotides generated from SEQ. ID. Nos: 1 to 5, useful for the identification of *C. glabrata.* In the lane 1 shows molecular weight marker. The lanes marked C1+ to C4+, are those with DNA from *C. glabrata.* The lanes marked C1- to C4- are those with DNA from S. *cerevisiae.* Lanes M1+ to M4 + containing DNA from *C. glabrata, C. albicans, C. kruzei, C. parapsilosis, C. tropicalis* and *C*. *guillermondii.* Lanes M1- to M4-containing DNA from *C. albicans, C. kruzei, C. parapsilosis, C. tropicalis* and *C. guillermondii.* Amplification bands positive for *C. glabrata* are 2205pb (samples C1 + and M1 +), 289pb (samples C2 + and M2 +), 196pb (samples C3 + and M3 +) and 410 bp (samples C4 + and M4 +). The molecular weight marker used is 1 Kb Plus DNA Ladder (Invitrogen).
Figure 3. 2% agarose gel, showing different pairs of oligonucleotides generated from SEQ. ID. Nos: 1 to 5, useful for the identification of *C. glabrata.* In the lane 1 shows molecular weight marker. Marked lanes C5 + to C8 +, are those with DNA from *C. glabrata.* The lanes marked C5- to C8-, are those with DNA from S. *cerevisiae.* Lanes M5+ to M8 + to contain DNA from *C. glabrata, C. albicans, C. kruzei, C. parapsilosis, C. tropicalis* and *C. guillermondii.* Lanes M 5 - to M8-containing DNA from *C. albicans, C. kruzei, C. parapsilosis, C. tropicalis* and *C. guillermondii.* Amplification bands positive for *C. glabrata* are 301 pb (samples C5 + and M5 +), 267pb (samples C6 + and M6 +), 130pb (samples C7 and M7 + +) and 428 bp (samples with C8 + and M8 +). The molecular weight marker used is 1 Kb Plus DNA Ladder (Invitrogen).

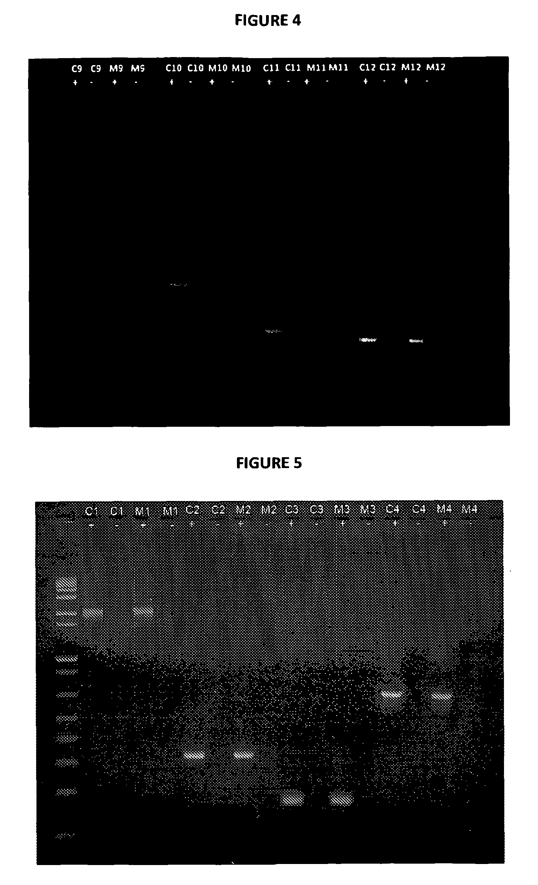

Figure 4. 2% agarose gel, showing different pairs of oligonucleotides generated from SEQ. ID. Nos: 1 to 5, useful for the identification of *C*. *glabrata.* In the lane 1 shows molecular weight marker. The lanes marked C9 + to C12 + are those with DNA from *C. glabrata.* The lanes marked C9- to C12 - are those with DNA from S. *cerevisiae.* Lanes M9 + to M12 + containing DNA from *C. glabrata, C. albicans, C. kruzei, C. parapsilosis, C. tropicalis* and *C. guillermondii.* Lanes M9- to M 12-containing DNA from *C. albicans, C. kruzei, C. parapsilosis, C. tropicalis* and C. *guillermondii.* Amplification bands positive for *C. glabrata* are 200pb (samples with C9 + and M9 +), 559pb (samples with C10 + and M10 +), 331 pb (samples with C11 + and M11 +) and 306 bp (samples with C 12 + and M12 +). The molecular weight marker used is 1 Kb Plus DNA Ladder (Invitrogen).
Figure 5. 2% agarose gel, showing different pairs of oligonucleotides generated from SEQ. ID. Nos: 1 to 5, useful for the identification of *C. glabrata.* In the lane 1 shows molecular weight marker. The lanes marked C1+ to C4, are those with DNA from *C. glabrata.* The lanes marked C1- to C4- are those with DNA from S. *cerevisiae.* Lanes M1+ to M4 + containing DNA from *C. glabrata, C. albicans, C. kruzei, C. parapsilosis, C.tropicalis* and *C. guillermondii.* Lanes M1- to M4-containing DNA from *C. albicans, C. kruzei, C. parapsilosis, C. tropicalis* and *C. guillermondii.* Amplification bands positive for *C. glabrata* are 2080pb (samples C1 + and M1 +), 332pb (samples C2 + and M2 +), 177pb (samples C3 + and M3 +) and 673 bp (samples C4 + and M4 +). The molecular weight marker used is 1 Kb Plus DNA Ladder (Invitrogen).

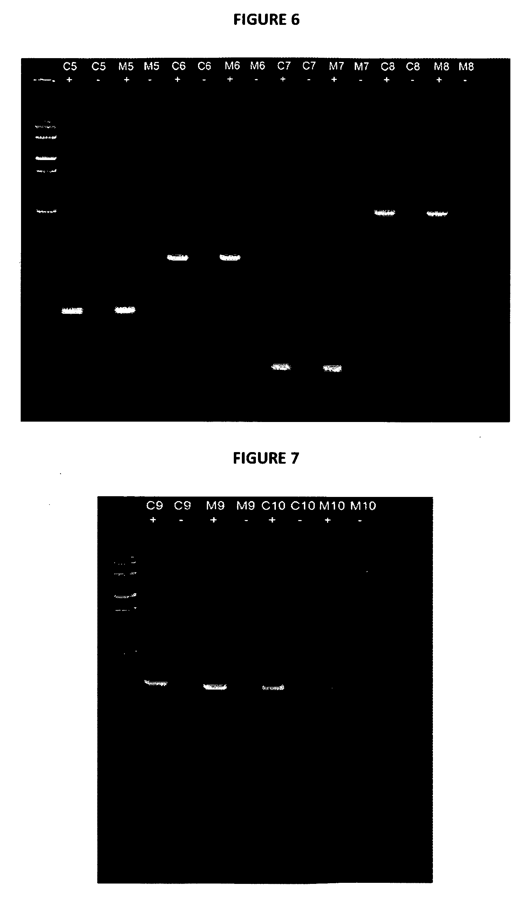

Figure 6. 2% agarose gel, showing different pairs of oligonucleotides generated from SEQ. ID. Nos: 1 to 5, useful for the identification of *C. glabrata.* In the lane 1 shows molecular weight marker. Marked lanes C5 + C8 +, are those with DNA from *C. glabrata.* The lanes marked C5- to C8-, are those with DNA from S. *cerevisiae.* Lanes M5+ to M8+ contain DNA from *C. glabrata, C. albicans, C. kruzei, C parapsilosis, C tropicalis* and *C guillermondii* Lanes M5- to M8-containing DNA from *C. albicans, C. kruzei, C. parapsilosis, C. tropicalis* and *C. guillermondii.* Amplification bands positive for C. *glabrata* are 339pb (samples C5 + and M5 +), 600pb (samples C6 + and M6 +), 162pb (samples C7+ and M7+) and 1004 bp (samples with C8 + and M8 +). The molecular weight marker used is 1 Kb Plus DNA Ladder (Invitrogen).
Figure 7. 2% agarose gel, showing different pairs of oligonucleotides generated from SEQ. ID. Nos: 1 to 5, useful for the identification of *C*. *glabrata.* In the lane 1 shows molecular weight marker. The lanes marked C9+ to C10 + are those with DNA from *C. glabrata.* The lanes marked C9- to C10 - are those with DNA from *S. cerevisiae.* Lanes M9+ to M10+ containing DNA of *C. glabrata, C. albicans, C. kruzei, C. parapsilosis, C.tropicalis* and *C. guillermondii.* Lanes M9- to M10-containing DNA from *C. albicans, C. kruzei, C. parapsilosis, C. tropicalis* and *C. guillermondii.* Amplification bands positive for *C*. *glabrata* are 740pb (samples with and M9+ and C9+) and 741pb (samples with C10+ and M10+). The molecular weight marker used is 1 Kb Plus DNA Ladder (Invitrogen).

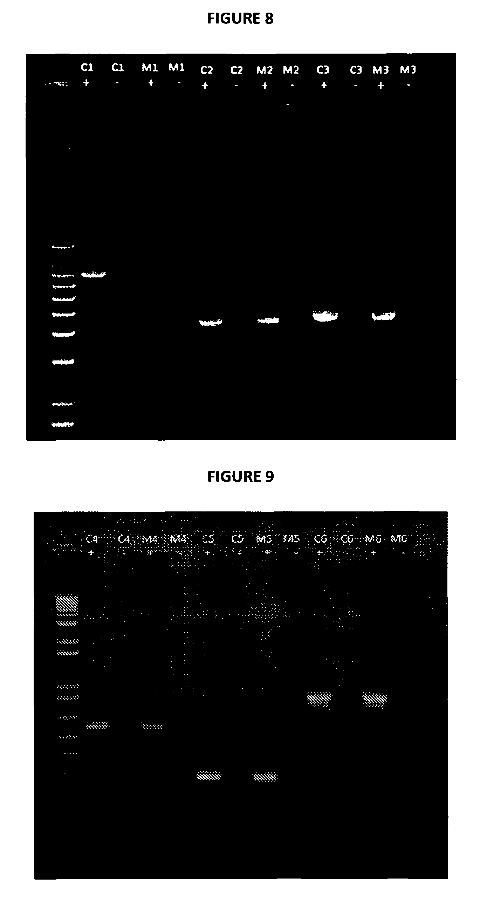

Figure 8. Gel 0.6% agarose showing different pairs of oligonucleotides generated from SEQ. ID. Nos: 1 to 5, useful for the identification of *C. glabrata.* In the lane 1 shows molecular weight marker. The lanes marked with C1+ to C3+ are those with DNA from *C. glabrata.* The lanes marked C1- to C3- are those with DNA from *S*. *cerevisiae.* Lanes M1+ to M3 + containing DNA from *C. glabrata, C. albicans, C. kruzei, C. parapsilosis, C.tropicalis* and *C. guillermondii.* Lanes MI- to M3-containing DNA from *C. albicans, C. kruzei, C. parapsilosis, C. tropicalis* and *C. guillermondii.* Amplification bands positive for *C. glabrata* are 8209pb (samples C1 + and M1 +), 4531 pb (samples C2 + and M2+) and 4596pb (samples C3+ and M3 +). The molecular weight marker used is 1 Kb Plus DNA Ladder (Invitrogen).
Figure 9. Gel 1.5% agarose showing different pairs of oligonucleotides generated from SEQ. ID. Nos: 1 to 5, useful for the identification of *C*. *glabrata.* In the lane 1 shows molecular weight marker. The lanes marked with C4 + to C6 + are those with DNA from *C. glabrata.* The lanes marked C4- to C6- are those with DNA from *S. cerevisiae.* Lanes M4 + to M6 + containing DNA from *C. glabrata, C. albicans, C. kruzei, C. parapsilosis, C. tropicalis* and *C. guillermondii.* Lanes M4- to M6-containing DNA from *C*. *albicans, C. kruzei, C. parapsilosis, C. tropicalis* and *C. guillermondii.* Amplification bands positive for *C. glabrata* are 593pb (samples C4 + and M4 +), 274pb (samples C5 + and M5 +) and 859 bp (samples C6 + and M6 +). The molecular weight marker used is 1 Kb Plus DNA Ladder (Invitrogen).

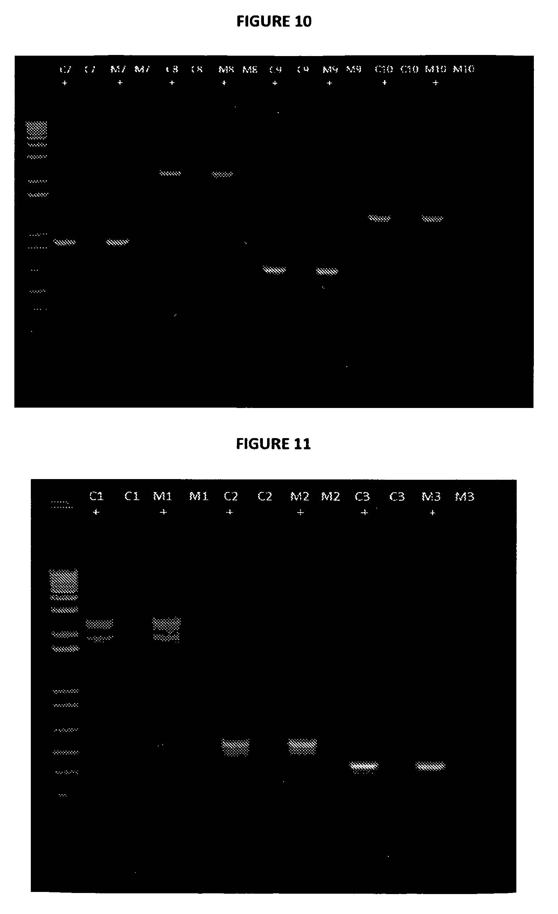

Figure 10. Gel 1.5% agarose showing different pairs of oligonucleotides generated from SEQ. ID. Nos: 1 to 5, useful for the identification of *C. glabrata.* In the lane 1 shows molecular weight marker. The lanes marked C7+ to C10+ are those with DNA from *C. glabrata.* The lanes marked with C7- to C10- are those with DNA from *S. cerevisiae.* Lanes M8+ to M10 + contain DNA of *C*. *glabrata, C. albicans, C. kruzei, C. parapsilosis,* C.tropicalis and *C. guillermondii.* Lanes marked with M7- to M10- containing DNA from *C. albicans, C. kruzei, C. parapsilosis, C. tropicalis* and *C. guillermondii.* Amplification bands positive for *C. glabrata* are 918pb (samples C7 + and M7 +), 2271 pb (samples with C8 + and M8 +), 689pb (samples with C9+ and M9+) and 1306 bp (samples with C10 + and M10 +). The molecular weight marker used is 1 Kb Plus DNA Ladder (Invitrogen).
Figure 11. Gel 1.5% agarose showing different pairs of oligonucleotides generated from SEQ. ID. Nos: 1 to 5, useful for the identification of *C. glabrata.* In the lane 1 shows molecular weight marker. The lanes marked with C1+ to C3+ are those with DNA from *C. glabrata.* The lanes marked C1- to C3- are those with DNA from *S. cerevisiae.* Lanes M1+ to M3 + containing DNA from *C. glabrata, C. albicans, C. kruzei, C. parapsilosis, C.tropicalis* and *C. guillermondii.* Lanes M1- to M3-containing DNA from *C. albicans, C. kruzei, C. parapsilosis, C. tropicalis* and *C. guillermondii.* Amplification bands positive for *C. glabrata* are 2412pb and 1940pb (samples C1+ and M1+), 556pb (samples C2+ and M2+) and 427pb (samples C3+ and M3+). The molecular weight marker used is 1 Kb Plus DNA Ladder (Invitrogen).

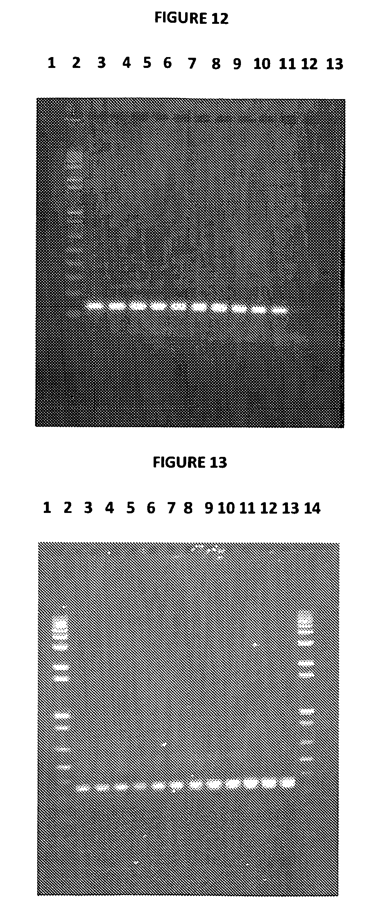

Figure 12. 2% agarose gel showing the optimization of temperature gradient for the PCR reaction with oligonucleotides SEQ ID Nos. 7 and 8, the identified bands are 136 and 140 bp. In the lane 1 shows molecular weight marker and lanes 2 to 13 show the PCR products at temperatures of 54.0, 54.9, 56.3, 57.6, 59.0, 60.3, 61.4, 62.9, 64.4, 65.8, 67.1 and 67.6 ° C respectively, corresponding to DNA of *C. glabrata,* which was checked by resequencing of PCR products. The molecular weight marker used is 1 Kb Plus DNA Ladder (Invitrogen).
Figure 13. 2% agarose gel showing the optimization of temperature gradient for the PCR reaction with oligonucleotides SEQ ID Nos. 6 and 8, the identified bands are 327, 348 and 364 bp. In lanes 1 and 14 shows the molecular weight marker and lanes 2 to 13 show the PCR products at temperatures of 54.0, 54.9, 56.3, 57.6, 59.0, 60.3, 61.4, 62.9, 64.4, 65.8, 67.1 and 67.6 °C respectively. The molecular weight marker used is 1 Kb Plus DNA Ladder (Invitrogen).

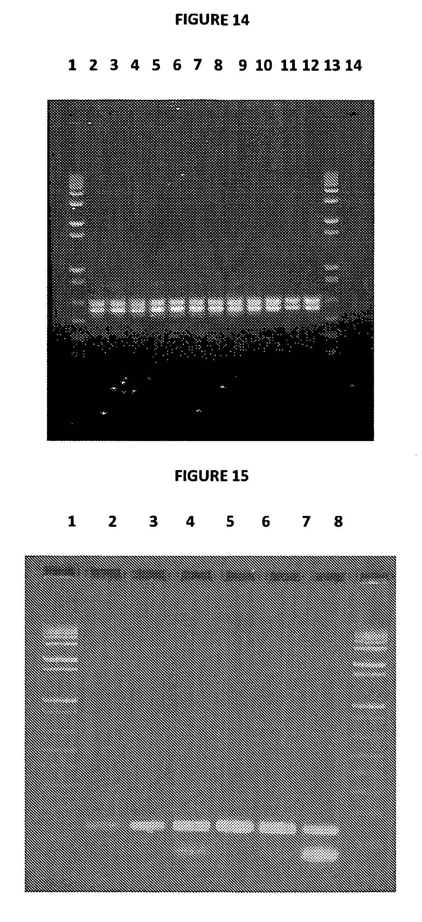

Figure 14. 2% agarose gel showing the optimization of temperature gradient for the PCR reaction with oligonucleotides SEQ ID Nos. 9 and 10, the identified bands are 601 and 664 bp. In lanes 1 and 14 shows the molecular weight marker and lanes 2 to 13 show the PCR products at temperatures of 54.0, 54.9, 56.3, 57.6, 59.0, 60.3, 61.4, 62.9, 64.4, 65.8, 67.1 and 67.6 °C respectively. The molecular weight marker used is 1 Kb Plus DNA Ladder (Invitrogen).
Figure 15. 2% agarose gel where the concentration is optimized for oligonucleotide primers of SEQ ID Nos. 7 and 8 at a temperature of 59 °C, where in lanes 1 and 8 are molecular weight markers and in lanes 2 to 7, the concentrations of primers are 100, 200, 400, 500, 600 and 800 nM. The PCR products obtained are 136 and 140 bp, which was verified by resequencing of PCR products. The molecular weight marker used is 1 Kb Plus DNA Ladder (Invitrogen).

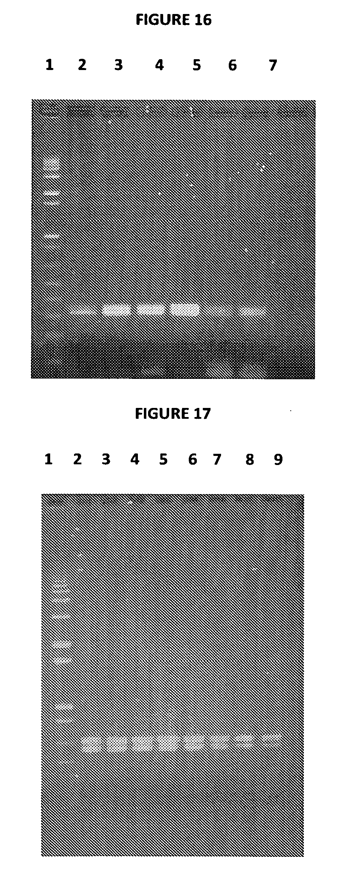

Figure 16. 2% agarose gel where the concentration is optimized for oligonucleotide primers of SEQ ID Nos. 6 and 8, at a temperature of 67.1 °C, where lane 1 shows molecular weight marker and lanes 2-7 concentrations of primers are 100, 200, 400, 500, 600 and 800 nM. The PCR products obtained are 327, 348 and 364 bp. The molecular weight marker used is 1 Kb Plus DNA Ladder (Invitrogen).
Figure 17. 2% agarose gel where the concentration is optimized for oligonucleotide primers of SEQ ID Nos. 9 and 10, at a temperature of 61.4 °C, where lane 1 shows molecular weight marker and lanes 2-9 concentrations of primers are 100, 200, 400, 500, 600, 800, 1000 and 1200 nM. The PCR products obtained are 601 and 664 bp. The molecular weight marker used is 1 Kb Plus DNA Ladder (Invitrogen).

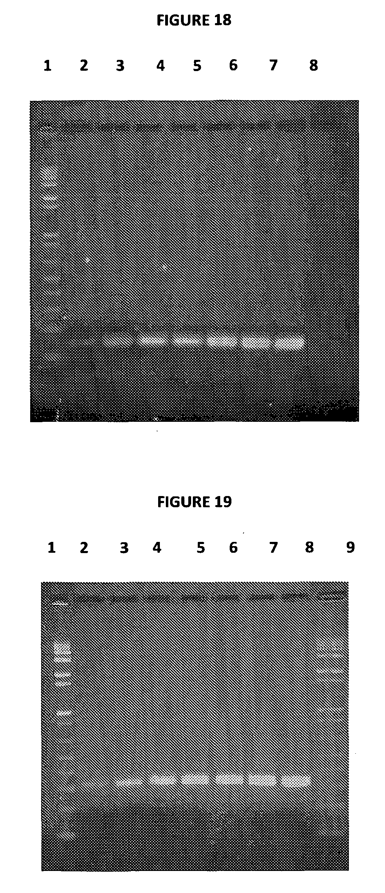

Figure 18. 2% agarose gel showing the optimization of the concentration of dNTPs for the oligonucleotides of SEQ ID Nos. 6 and 8 at a temperature of 59°C and a primer concentration of 500 nM. In the lane 1 shows molecular weight marker, and lanes 2 to 8 show the concentrations of 5, 10, 15, 20, 30, 40 and 50 µM dNTPs. The PCR products obtained are 136 and 140 bp. The molecular weight marker used is 1 Kb Plus DNA Ladder (Invitrogen).
Figure 19. 2% agarose gel showing the optimization of the concentration of dNTPs for the oligonucleotides of SEQ ID Nos. 7 and 8, at a temperature of 67.1 °C and a primer concentration of 200 nM. In the lane 1 shows molecular weight marker, and lanes 2 to 8 show the concentrations of 5, 10, 15, 20, 30, 40 and 50 µM dNTPs. The PCR products obtained are 327, 348 and 364 bp. The molecular weight marker used is 1 Kb Plus DNA Ladder (Invitrogen).

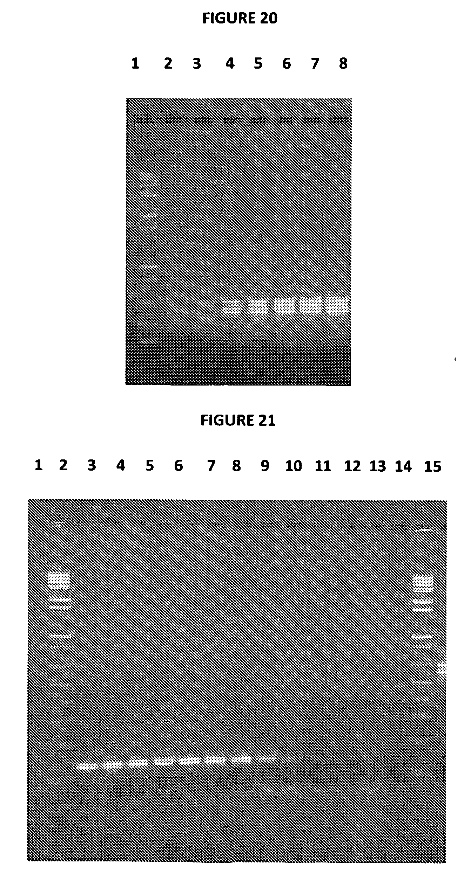

Figure 20. 2% agarose gel showing the optimization of the concentration of dNTPs for the oligonucleotides of SEQ ID Nos. 9 and 10, at a temperature of 61.4 °C and a primer concentration of 400 nM. In the lane 1 shows molecular weight marker, and lanes 2 to 8 show the concentrations of 5, 10, 15, 20, 30, 40 and 50 µM dNTPs. The PCR products obtained are 601 and 664 bp. The molecular weight marker used is 1 Kb Plus DNA Ladder (Invitrogen).
Figure 21. 2% agarose gel which shows the sensitivity of the PCR reaction by varying the amount of genomic DNA from the sample to the oligonucleotides of SEQ ID Nos. 6 and 8, at a temperature of 59°C, a primer concentration of 500nM, dNTPs concentration of 30µM. In lanes 1 and 15 are molecular weight markers, and lanes 2 to 14 show the concentrations of 345, 200, 20, 12.5, 2.5, 0.5, 0.1, 0.02, 0.004, 0.0008, 0.00016, 0.000032 and 0 ng of genomic DNA. The PCR products obtained are 136 and 140 bp. The molecular weight marker used is 1 Kb Plus DNA Ladder (Invitrogen).

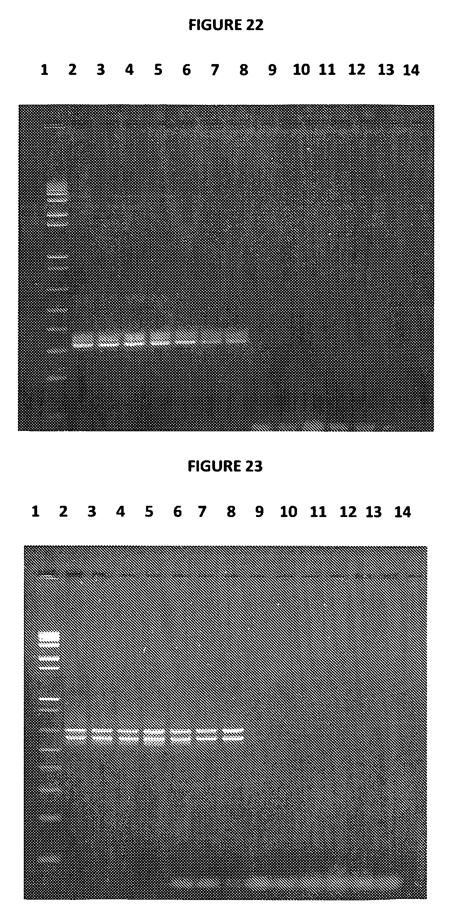

Figure 22. 2% agarose gel which shows the sensitivity of the PCR reaction by varying the amount of genomic DNA from the sample to the oligonucleotides of SEQ ID Nos. 7 and 8, at a temperature of 67.1°C, a primer concentration of 200nM, dNTPs concentration of 30µM. In the lane 1 shows molecular weight marker, and lanes 2 to 14 show the concentrations of 345, 200, 20, 12.5, 2.5, 0.5, 0.1, 0.02, 0.004, 0.0008, 0.00016, 0.000032 and 0 ng genomic DNA. The PCR products obtained are 327, 348 and 364 bp. The molecular weight marker used is 1 Kb Plus DNA Ladder (Invitrogen).
Figure 23. 2% agarose gel which shows the sensitivity of the PCR reaction by varying the amount of genomic DNA from the sample to the oligonucleotides of SEQ ID Nos. 9 and 10, at a temperature of 61.4°C, a primer concentration of 400nM, dNTPs concentration of 30µM. In the lanes 1 and 15 are molecular weight markers, and lanes 2 to 14 show the concentrations of 345, 200, 20, 12.5, 2.5, 0.5, 0.1, 0.02, 0.004, 0.0008, 0.00016, 0.000032 and 0 ng genomic DNA. The PCR products obtained are 601 and 664 bp. The molecular weight marker used is 1 Kb Plus DNA Ladder (Invitrogen).

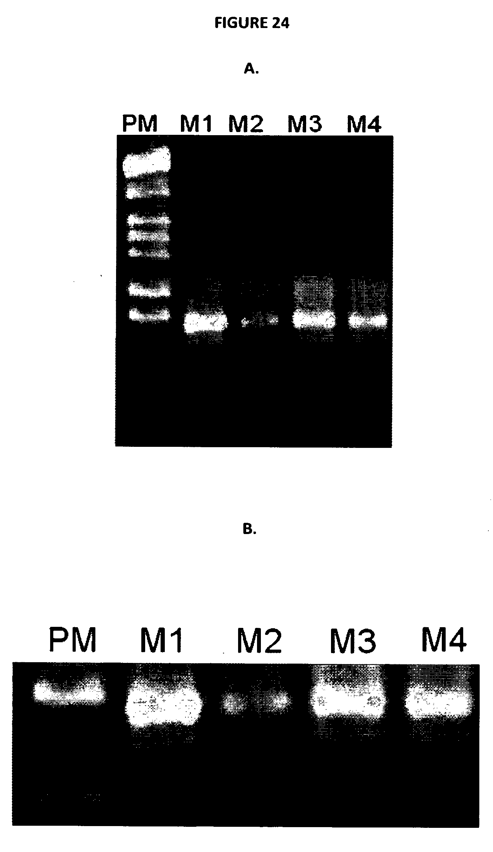

Figure 24. 6% native polyacrylamide gel. Gel A: lane PM shows the DNA molecular weight marker pBR322/Msp I (biogenic) in lanes 2 to 5 show the PCR reaction products obtained at 59°C, 500 nM primers and 30µM dNTPs with the oligonucleotides of SEQ. ID. Nos. 7 and 8, where the bands obtained are 136 and 140 base pairs. Gel B: A close-up of gel A: lane PM: bands of 147 and 123 base pairs pBR322/Msp molecular weight marker I (biogenic), lanes 2 to 5 correspond to the bands of 136 and 140 base pairs of PCR products obtained using oligonucleotides of SEQ. ID. Nos. 7 and 8.

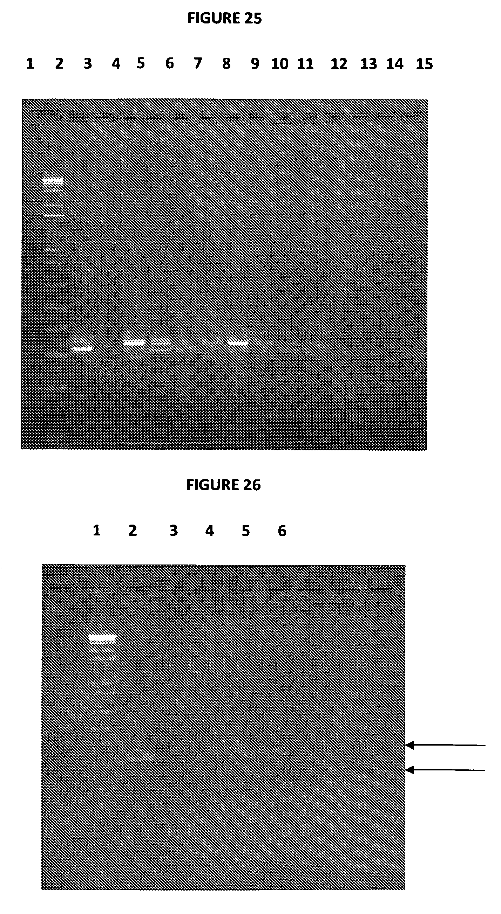

Figure 25. 2% agarose gel, which shows the sensitivity and specificity of the PCR reaction by using clinical samples. Oligonucleotides of SEQ ID Nos. 6 and 8 were used. In lane 1 is molecular weight marker, lane 2, a sample of genomic DNA from a wild-type strain of *C. glabrata,* lane 3, a sample of genomic DNA of a wild-type strain of *C. albicans,* in lanes 4 to 15 clinic urine samples. The molecular weight marker used is 1 Kb Plus DNA Ladder (Invitrogen).
Figure 26. 2% agarose gel, which shows the sensitivity and specificity of the PCR reaction in clinical samples of blood. In lane 1 is molecular weight marker, lane 2, genomic DNA from a wild-type strain of *C. glabrata,* lane 3, genomic DNA from a wild-type strain of *C. albicans.* In lane 4, a blood sample negative for *C. glabrata,* in lanes 5 and 6, two genomic DNA samples positive for *C. glabrata.* The PCR products are 327, 348 and 364 bp. The molecular weight marker used is 1 Kb Plus DNA Ladder (Invitrogen).

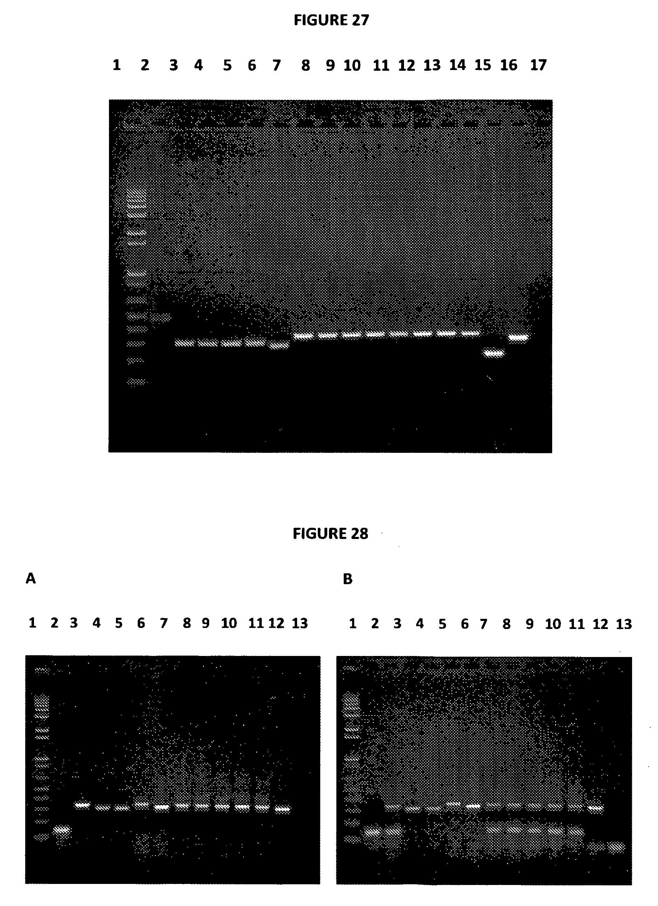

Figure 27. Agarose gel 2%, which shows that the negative element is found only in *C. glabrata* and not in other phylogenetically related species. Several probes were used, the oligonucleotides of SEQ ID Nos. 9 and 10 to the negative element of *C. glabrata* in lanes 2 to 17 and specific oligonucleotide 26 S rRNA in lanes 3 to 7, specific oligonucleotide 18 S rRNA in lanes 8 to 17. The molecular weight marker used is 1 Kb Plus DNA Ladder (Invitrogen).
Figure 28. Agarose gel 2%, which demonstrates the specificity of the oligonucleotides generated from SEQ. ID. Nos. 1 to 5 in mixtures of *C. glabrata* with phylogenetically close species. In gel A, lane 2, oligonucleotides with SEQ ID Nos. 7 and 8 were used, lanes 3 to 13 using rRNA oligonucleotide, the gel B, lanes 2 to 13 using the oligonucleotides of SEQ. ID. Nos. 7 and 8 and lanes 3 to 13 are also using rRNA oligonucleotide. In lanes 1 to 13 of each gel the following samples were run in this order: lane 1, molecular weight marker, Lane 2, negative element of *C. glabrata,* lane 3, *C. glabrata,* lane 4, *C*. *parapsilosis;* lane 5, *C. tropicalis;* lane 6, *C. parapsilosis;* lane 7, *C*. *albicans,* lanes 8 to 13, mix of *C.albicans* (100 ng) with C. *glabrata* at concentrations of 345, 20, 10, 5, 2.5 and 0 ng of *C. glabrata..* Amplification bands were observed for rRNA in lanes 3 to 13 and only in lanes 2, 3 and 8 to 13, 136 and 140 bp for *C. glabrata.* The molecular weight marker used is 1 Kb Plus DNA Ladder (Invitrogen).

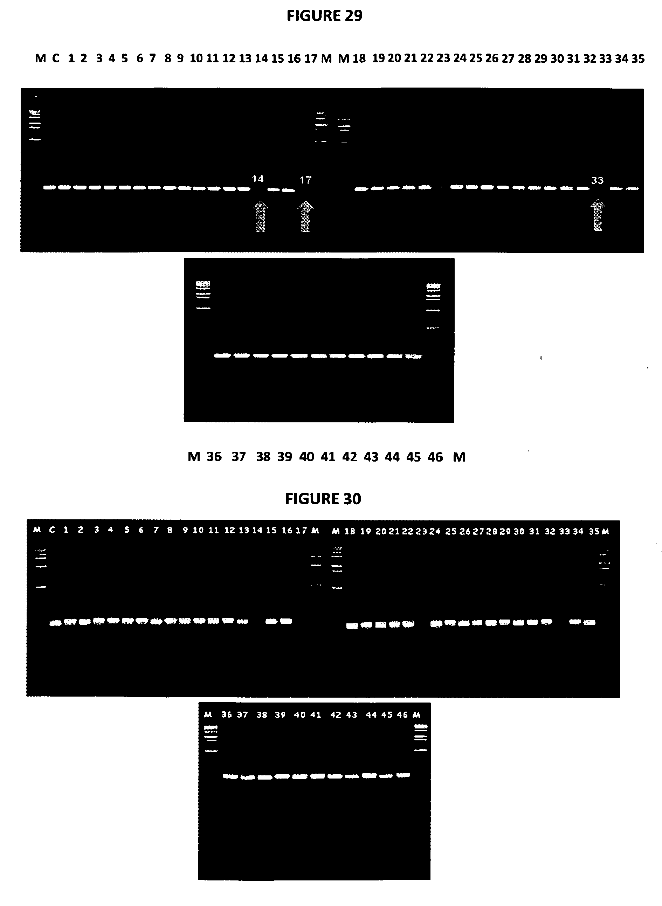

Figure 29. Agarose gel 2%, which identifies *C. glabrata* from different clinical isolates. In lanes M are molecular weight markers. Lane C, shows the positive control of *C. glabrata.* In lanes 1 to 46 show various clinical isolates. Lanes 14, 17 and 33 show no amplification band, which will be explained in the examples section. The molecular weight marker used is 1 Kb Plus DNA Ladder (Invitrogen).
Figure 30. Agarose gel 2%, which identifies *C. glabrata* from different clinical isolates. In lanes M are molecular weight markers. Lane C, shows the positive control of *C. glabrata.* In lanes 1 to 46 show various clinical isolates. Lanes 14, 17 and 33 show no amplification band, which will be explained in the examples section. The molecular weight marker used is 1 Kb Plus DNA Ladder (Invitrogen).

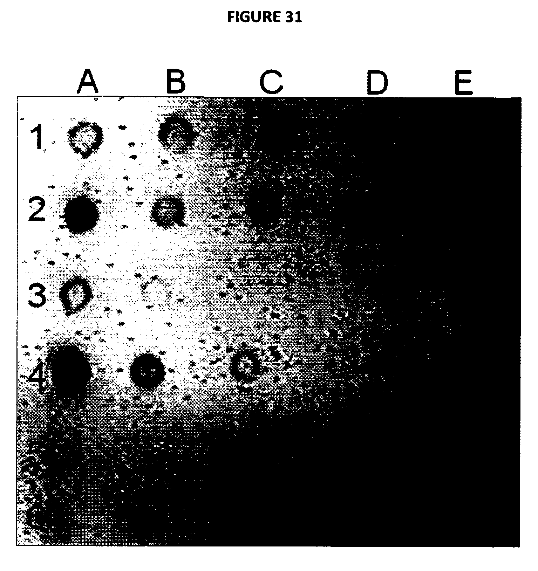

Figure 31. Use of a DNA molecule as a hybridization probe for specific identification of *C. glabrata.* This figure shows a Dot-blot analysis, where the row 1 shows a PCR product as an internal control, varying amounts of DNA from 50, 40, 30, 20 and 10 ng in lanes A to E respectively. In row 2 shows DNA from a clinical sample of *C. glabrata* in quantities of 10, 5, 2.5, 1 and 0.5 mg in lanes A to E, respectively. In row 3 shows DNA from a clinical sample of *C. glabrata* in quantities of 5, 2.5, 1, 0.5 and 0.25 mg in lanes A to E, respectively. In row 4 shows the DNA of a strain of *C. glabrata* in quantities of 10, 5, 2.5, 1 and 0.5 mg in lanes A to E, respectively. In row 5 shows the DNA of a strain of *C. albicans* in quantities of 10, 5, 2.5, 1 and 0.5 mg in lanes A to E, respectively. In row 6 shows the DNA of a strain of *Saccharomyces cerevisiae* in quantities of 10, 5, 2.5, 1 and 0.5 mg in lanes A to E, respectively. The *C. glabrata* probe used is 140 bp and is located in the intergenic region of SEQ. ID. Nos. 1 to 3.

### BRIEF DESCRIPTION OF THE INVENTION

The present invention describes and claims a DNA molecule, wherein is an intergenic region of adhesin *C. glabrata* and is selected from the group consisting of SEQ ID No. 1, SEQ ID No. 2, SEQ ID No. 3, SEQ ID No. 4 and SEQ ID No. 5.

In a further embodiment, said DNA molecule is comprised of the following intergenic regions: EPA1 to EPA 2 for SEQ ID No. 1 (2225 bp), EPAH to Telomere for SEQ. ID No. 2 (2438 bp), EPAK to EPA22 for SEQ. ID. No. 3 (8309 bp), EPA6 to Telomere for SEQ. ID. No. 4 (2432 bp) and EPA7 to Telomere for SEQ. ID. No. 5 (2498 bp).

It also describes and claims an oligonucleotide for specific identification of *C. glabrata,* characterized in that it consists of a continuous sequence of 15 to 35 nucleotides of the DNA sequences selected from the group consisting of SEQ ID No. 1, SEQ ID No. 2, SEQ ID No. 3, SEQ ID SEQ ID No. 4 and No. 5., wherein said oligonucleotide is further characterized as consisting of sequences shown in SEQ ID Nos. 6 to 52. This oligonucleotide is further characterized because it has a homology between 90 and 100% with respect to SEQ ID Nos. 1 to 5.

In a further aspect of the present invention it is described and claimed an in vitro method for the specific identification of *C. glabrata,* which comprises the steps of: a) amplifying DNA fragments from a biological sample by PCR with the oligonucleotides described above and b) identify the amplified DNA fragments. In this method the biological sample is derived from one subject to study. Wherein the study subject is a mammal, which in a preferred embodiment, but not limited is a human. In a further preferred embodiment, said biological sample is selected from the group consisting of any sample containing DNA, fluids, tissue, or cell debris, midstream urine, urine culture tube, growing by nephrostomy (right and left kidney), hemodialysis water, pleural fluid, pyogenic culture, mieloculture, bone marrow, blood lysis (peripheral blood), blood culture (blood), leukocyte concentrate, concentrated red cell, throat, nasal discharge, vaginal discharge, prostate exudates, sputum, catheter, biopsies from different tissues such as lymph node, subcutaneous tissue, cornea, lung, pulmonary nodule, pancreas, jaw, skin, quantitative skin (cellulite, breast, scrotum, arm, hand), hair, nails, shinbone, muscle, bone, breast, synovial fluid, scar, thigh, joint capsule, knee, omentum, bronchoalveolar lavage (lingula, upper and lower lobe (left and right), left and right LBA (airways)); post-mortem (liver, lung, spleen), wound swabs (perianal, vaginal, ulcer (foot, hand)), abscess (thigh, kidney, perianal) or peripancreatic.

In a further embodiment of the method, the amplified DNA fragments were carried out with at least one oligonucleotide as defined above. Also, in preferred embodiments of the method described herein and claimed, the temperature reaction amplification of PCR is between 54°C and 67.6°C, the concentration of genomic DNA is equal to or greater than 0.0008ng, preferably the concentration of DNA genome is between 0.0008ng to 345ng, in addition, the concentration of oligonucleotide is between 100nM and 1200nM and the concentration of dNTPs is between 5 to 50 µM.

In a further embodiment of the present invention describes and claims a kit for the specific identification of *Candida glabrata,* which comprises the oligonucleotides as defined above. In a preferred embodiment, said kit comprises at least one sample of genomic DNA from *C. glabrata* (positive control) and at least one sample of genomic DNA from at least one species phylogenetically related to *C. glabrata* (negative control).

In a preferred embodiment, it is described and claimed the use of a DNA molecule that is an intergenic region of adhesion from *C. glabrata* and is selected from the group consisting of SEQ ID No. 1, SEQ ID No. 2, SEQ ID No. 3, SEQ ID No. 4 and SEQ ID No. 5, as described above, to generate oligonucleotides useful for specific identification of *C. glabrata.*

Additionally, claims and describes the use of at least two oligonucleotides characterized because they consist of a continuous sequence of 15 to 35 nucleotides of the DNA sequences selected from the group consisting of SEQ ID No. 1, SEQ ID No. 2, SEQ ID No. 3, SEQ ID No. 4 and SEQ ID No. 5. Wherein such oligonucleotides are further characterized as consisting of the sequences as shown in SEQ ID Nos. 6 to 52. Said oligonucleotides are further characterized as having a homology between 90 and 100% with respect to SEQ ID Nos. 1 to 5, to identify *C. glabrata.*

A preferred embodiment of the present invention comprises a DNA molecule useful for specific identification of *C*. *glabrata,* characterized in that it consists of a continuous sequence of 36 or more nucleotides comprised within DNA sequences are selected from the group consisting of SEQ ID No. 1, SEQ ID No. 2, SEQ ID No. 3, SEQ ID No. 4 and SEQ ID No. 5, wherein said molecule has a homology between 90 and 100% with respect to SEQ ID Nos. 1 to 5, as well as their use.

Within the scope of the present invention it is described and claimed an in vitro method for the specific identification of *C*. *glabrata,* which comprises the steps of: a) hybridizing DNA fragments from a biological sample with the DNA molecules as defined in the previous paragraph b) identifying the hybridized DNA fragments, wherein steps a) and b) are carried out by a technique selected from the group comprising: Dot-Blot, Southern blotting, Northern blotting, in situ hybridization or microarray, wherein the biological sample is derived from one subject to study, wherein the subject to study is a mammal, wherein said mammal is a human. Said biological sample is selected from the group consisting of any sample containing DNA, fluids, tissue, or cell debris, midstream urine, urine culture tube, growing by nephrostomy (right and left kidney), hemodialysis water, pleural fluid, pyogenic culture, mieloculture, bone marrow, blood lysis (peripheral blood), blood culture (blood), leukocyte concentrate, concentrated red cell, throat, nasal discharge, vaginal discharge, prostate exudates, sputum, catheter, biopsies from different tissues such as lymph node, subcutaneous tissue, cornea, lung, pulmonary nodule, pancreas, jaw, skin, quantitative skin (cellulite, breast, scrotum, arm, hand), hair, nails, shinbone, muscle, bone, breast, synovial fluid, scar, thigh, joint capsule, knee, omentum, bronchoalveolar lavage (lingula, upper and lower lobe (left and right), left and right LBA (airways)); post-mortem (liver, lung, spleen), wound swabs (perianal, vaginal, ulcer (foot, hand)), abscess (thigh, kidney, perianal) or peripancreatic. Also, the step a) is performed with at least one DNA molecule as defined above.

Finally, within the scope of this application is a kit for the specific identification of *Candida glabrata,* further comprising at least one DNA molecule as defined above, wherein said kit comprises at least one sample of genomic DNA from C. glabrata (positive control) and at least one sample of genomic DNA from at least one species phylogenetically related to *C. glabrata* (negative control).

### DETAILED DESCRIPTION OF THE INVENTION

### Sequences and diagnostic probes obtaining.

The sequences described and claimed herein are included within the genomic DNA sequence of *C. glabrata* (Http://cbi.labri.fr/Genolevures/elt/CAGL/) and GeneBank sequences Nos. AY344225, AY344226, AY646926, AY646925. However, without prejudice to the patentability of the invention, its characterization as cis-regulatory sequences of the adhesins associated with these sequences and their use as useful for identification of *C. glabrata* has not been described in any scientific paper or patent databases.

However, the regulatory sequences of genes for adhesins subject of this application are related to the regulation of expression, for example, the adhesin EPA1. It has been observed that regulation of the adhesin in vitro is complex: the gene is repressed in cultured cells in stationary phase and is strongly induced while diluted in fresh medium. During the exponential phase, expression is repressed by the action of a negative element (EN).The EN is an element in cis, and initially functionally mapped the 3 'end of the TAA of EPA1. By bioinformatic analysis it has been found that the functionally mapped element is found only in *C. glabrata* and at blocks, which can vary depending on the analyzed serotype. The genome of *C. glabrata* has 5 copies of the EN (including the original 3' end of EPA1), associated with any of the EPA gene family. The detection of EN by oligonucleotide generated along the sequence of these regions is essential for the identification of *C*. *glabrata.* Under the above, the sequences identified as SEQ ID Nos. 1 to 5 comprise the intergenic regions with blocks of negative elements, and therefore, any oligonucleotide generated from these sequences is useful for identification of *C. glabrata.* In this vein, the SEQ ID No. 1 (2225 bp) corresponds to the intergenic region between genes EPA1 and EPA2; SEQ ID No. 2 (2438 bp) corresponds to the intergenic region between gene EPA-H and the telomere; the SEQ ID No. 3 (8309 bp) corresponds to the intergenic region between genes EPAK and EPA-22; SEQ ID No. 4 (2432 bp) corresponds to the intergenic region between gene EPA6 and the telomere, and SEQ ID No. 5 (2498 bp) corresponds to the intergenic region between the gene EPA7 and the telomere. By way of example and without limitation to the scope of the present invention, SEQ. ID. Nos. 6 to 52 are shown, derived from SEQ. ID. Nos. 1 to 5, which allow the identification 100% specific and sensitive of clinical isolates of *C*. *glabrata* from different backgrounds. The relative alignment of SEQ. ID. Nos. 6 to 52 on SEQ. ID. Nos. 1 through 5 are represented in Figure 1.

Within the scope of the present invention will also include the complementary sequences at least 90% with SEQ. ID. Nos. 1 to 5.

In order to verify that any portion of the sequences described in SEQ. ID. Nos. 1 through 5 are useful for identifying *C. glabrata,* oligonucleotides were generated as described in SEQ. ID. Nos. 6 to 52, which cover all intergenic regions of adhesins EPA1, EPA22, EPAH, EPA6 and EPA7. The position of these oligonucleotides is shown in Figure 1, where one can see that these oligonucleotides were generated from the ends to the central regions. Also, it has been proved its usefulness for the identification of *C. glabrata* alone or in mixtures with other phylogenetically related species. Figures 2 to 11 show different combinatorial obtainable from the oligonucleotides of SEQ ID Nos. 6 to 52 and sizes of expected PCR product. The results show that all of the SEQ. ID. Nos. 1 to 5 serve as the basis for the generation of oligonucleotides specific for *C. glabrata,* maintaining sensitivity and specificity of the test, and it is possible to detect the fungus in a mixture with other yeasts. Therefore, any oligonucleotide generated from SEQ. ID. Nos. 1 to 5 falls within the scope of this application.

### Collection of biological samples.

Biological samples are from a subject of study, which is an animal, mammal, and in a preferred embodiment, said mammal is a human. Said samples can come from any biological fluid, tissue, or cell debris containing DNA in good condition to carry out PCR. Such samples can be selected, but are not limited to: midstream urine, urine culture tube, growing by nephrostomy (right and left kidney), hemodialysis water, pleural fluid, pyogenic culture, mieloculture, bone marrow, blood lysis (peripheral blood), blood culture (blood), leukocyte concentrate, concentrated red cell, throat, nasal discharge, vaginal discharge, prostate exudates, sputum, catheter, biopsies from different tissues such as lymph node, subcutaneous tissue, cornea, lung, pulmonary nodule, pancreas, jaw, skin, quantitative skin (cellulite, breast, scrotum, arm, hand), hair, nails, shinbone, muscle, bone, breast, synovial fluid, scar, thigh, joint capsule, knee, omentum, bronchoalveolar lavage (lingula, upper and lower lobe (left and right), left and right LBA (airways)); post-mortem (liver, lung, spleen), wound swabs (perianal, vaginal, ulcer (foot, hand)), abscess (thigh, kidney, perianal) or peripancreatic.

### In vitro diagnosis method of Candida glabrata by PCR.

In general, the method of the present invention comprises the following steps:
- Isolate DNA from a biological sample,
- Perform a PCR reaction using oligonucleotides generated from SEQ ID Nos. 1 to 5, including but not limited to the oligonucleotides of SEQ. ID.Nos. 6 to 52.
- Corroborate the presence of *Candida glabrata* by a conventional method.

These conventional methods are as described, but not limited to: Ausubel FM et al. (1999) Short protocols in molecular biology: a compendium of methods from Current protocols in molecular biology. John Wiley & Sons; Fasmann GD (1989) Practical handbook of biochemistry and molecular biology. Florida CRC Press, 1989; Sambrook J, Fritsch EF, Maniatis T (1989) Molecular cloning: a laboratory manual. Cold Spring Harbor Laboratory Press., Innis MA et al. (1990); PCR protocols: a guide to methods and applications. Academic Press; Innis MA, Gelfand DH, Sninsky JJ (1999) PCR applications: protocols for functional genomics. Academic Press; McClelland M, Honeycutt R, Mathieu-Daude F, Vogt T, Welsh J (1997) Fingerprinting by arbitrarily primed PCR. Methods Mol Biol. 85:13-24; McClelland M, Welsh J (1994) DNA fingerprinting by arbitrarily primed PCR. PCR Methods Appl. 4:S59-65; McPherson MJ, Hames BD, Taylor GR (1995) PCR 2 :a practical approach. IRL Press; McPherson MJ, Quirke P, Taylor GR (1994) PCR : a practical approach. Oxford University Press; Evans IH (1996) Yeast protocols: methods in cell and molecular biology. New Jersey Humana Press.

The identification of *C. glabrata* is carried out with 100% specificity and 100% sensitivity, because the sequences SEQ ID Nos. 1 to 5 are unique to the fungus and are not present in any other organism, and this is the first report about its usefulness for the identification of *C*. *glabrata.*

To prove the efficiency and specificity of the identification method of the present invention, the following experiments are displayed:
In order to demonstrate the standardization of experimental conditions for the specific identification of *C*. *glabrata,* the oligonucleotides described in SEQ ID Nos. 6 to 10 were chosen to be tested under the following conditions:
   a) Optimization of the temperature gradient: the oligonucleotides of SEQ ID Nos. 6 to 10 were tested in a temperature range between 54°C and 67.6°C. As shown in Figures 12 to 14, said oligonucleotides produced the expected PCR fragments of 136, 140, 327, 348, 364, 601 and 664 bp. This means that the reaction temperature may vary between 54°C and 67.6°C without changing the reaction product, its specificity and sensitivity.
   b) Optimization of the number of primers: in Figures 15 to 17 it is shown that the oligonucleotides of SEQ ID Nos. 6 to 10, were tested at concentrations of 100 to 1200 nM, and at temperatures between 59°C to 67.1°C, producing fragments of the expected size in all cases.
   c) dNTPs concentration gradient: in Figures 18 to 20 it is shown the optimization of the concentration of dNTPs at different temperatures between 59°C and 67.1°C and a concentration of primers from 200nM to 500nM. The concentration of dNTPs is between 5 and 50 µM, and in all cases PCR products of expected size are obtained.
   d) Concentration of genomic DNA: in figures 21 to 23 it is shown the minimum concentration required for amplification of genomic DNA from clinical samples. We used different pairs of oligonucleotides described in SEQ ID Nos. 6 to 10, temperatures between 59°C to 67.1°C, at a concentration of primers from 200nM to 500nM and a concentration of 30µM dNTPs. The concentrations of genomic DNA producing PCR products are from 0.0008ng to 345 ng.
   e) Resolution of PCR products of 136 and 140 bp oligos corresponding to SEQ ID NO: 7 and 8. Since in the agarose gel 2% used daily for the identification of PCR products is not possible to distinguish differences between some pairs of bands with very similar sizes, in the case of PCR products obtained for the pair of oligonucleotides SEQ ID Nos. 7 and 8 the presence of the two products was checked both by resequencing and by native acrylamide gel. Thus, to verify that indeed only two bands of 136 and 140 corresponding to the PCR products of the oligonucleotides of SEQ ID Nos. 7 and 8 were obtained, a native polyacrylamide gel to 6% was performed and stained with ethidium bromide. As shown in Figure 24, at different concentrations of DNA (from a DNA concentration of 345 ng in 30 microliters of final reaction and loaded in lanes M1 6 microliters, M2 3 microliters, M3 5 microliters and M4 4 microliters in said volume of 30 microliters), the presence of two bands of 136 and 140 base pairs expected is seen.

Standardization of experimental conditions for the diagnosis of *C. glabrata* shows that a wide variety of pairs of primers designed from SEQ. ID. Nos. 1 to 5 can be used, and by adjusting the conditions of for example, concentration of genomic DNA, oligonucleotides, dNTPs and PCR reaction temperature, an excellent resolution of the PCR products of expected sizes for each sequence is provided, and as seen in Figures 2 to 11 and later in Figures 13 and 18. Said identification is specific and sensitive for different isolates of *C. glabrata* from clinical isolates either alone or in combination with other species of fungi.

### Comparative analysis of traditional diagnostic method vs. the detection method of the present invention.

In order to fully test the competitive advantage of the methods of the present invention against traditional diagnostic methods, below is a comparison of the times of both tests:
Traditional method of identification of *Candida glabrata* in urine samples: urine samples are analyzed in an automated urine analyzer Urisys type coupled to UF-100i. The analysis was performed by flow cytometry with an argon laser. The UF-100i measures the properties of scattered light and fluorescence to count and identify the particles in the urine. The volume of the particles is determined from the impedance signals. Thus, according to the scatterplots, the result indicates which urine samples are likely to contain yeast like cells. These samples are marked as YLC urine samples (yeast like cells). In urine samples marked as YLC, 1µl is taken and plated on Sabourand / Dextrose medium (SDA) and Sabourand / Dextrose medium with cefoperazone (CFP). These plates are incubated at 30°C for 72 hours. Urine cultures with growth less than 10,000 CFU / ml, as well as plates withouth growth, are reported as not developed fungi (negative); urine cultures with equal or greater development to 10,000 CFU / ml pass to germ tube test, with incubation for 2 hours at 35°C, where they are reported as positive for *C. albicans* and *C*. *dubliniensis.* In the case of negative germ tube, it is reported as *Candida sp.* To identify the species from the report of *Candida sp.,* Vitek cards are used allowing the identification by means of carbohydrates assimilation. These cards are incubated for a period of 24 to 48 hours, at which time the cards are read. The minimum total time to identify *C. glabrata* is 6 days, with a sensitivity of about 85%.

Method of identifying *C. glabrata* of the present invention: the urine samples are analyzed in an automated urine analyzer URISYS type coupled to UF-100i. The analysis was performed by flow cytometry with an argon laser. The UF-100i measures the properties of scattered light and fluorescence to count and identify the particles in the urine. The volume of the particles is determined from the impedance signals. Thus, according to the scatterplots, the result indicates which urine samples are likely to contain yeast cells. These samples are marked as YLC urine samples (yeast-form cells). The time of this first stage is 2 hours. Next, in urine samples marked as YLC, 1 ml is taken, centrifuged, the supernatant is discarded, resuspended and the pellet boiled. The genomic DNA obtained is tempate for the PCR using primers generated from SEQ ID Nos. 1 to 5, such as but not limited to those described in SEQ ID Nos. 6 to 52, under optimal reaction conditions. The PCR products were separated by agarose gel electrophoresis and the products are analyzed for the correct identification of *C. glabrata.* The total test time is 6 hours.

Traditional method of identification of *Candida glabrata* in blood samples: Blood samples are incubated for 72 hours in the automated equipment BACTEC9240. When there is growth of microorganisms, these metabolize the nutrients in the culture medium by releasing CO₂. The release of CO₂ is detected by the computer and automatically marked as blood cultures positive for yeast. Positive blood cultures for yeasts are grown on plates Sabourand / Dextrose (SDA) and Sabourand / Dextrose with cefoperazone (CFP) and incubated at 30°C for 72 hours. Blood cultures with lower growth of 10,000 CFU / ml as well as those without growth, are reported as "Not Developed Fungus" (negative), on the blood cultures with growth equal to or greater than 10,000 CFU / ml germ tube test was performed for 2 hours at 35°C and is reported as positive for *C. albicans* and *C. dubiniensis.* In the case of negative germ tube is reported as *Candida sp.* To identify the species from the report of *Candida sp.,* Vitek cards are used allowing the identification by means of carbohydrates assimilation. These cards are incubated for 24 to 48 hours and are read to identify *C. glabrata.* The total time for identification is a minimum of 9 days.

Method to detect *C. glabrata* of the present invention in blood samples: Blood samples are incubated for 72 hours in a BACTEC9240 automated equipment. When there is microorganisms growth, these metabolize the nutrients in the culture medium by releasing CO₂. The release of CO₂ is detected by the computer and automatically marked as blood cultures positive for yeast. From blood samples marked as positive for yeast, 100µl are taken, centrifuged, the supernatant is discarded, resuspended and the pellet boiled. The genomic DNA obtained is the template for PCR reaction where any of the oligonucleotides generated from SEQ ID Nos. 1 to 5, such as but not limited to those described in SEQ ID Nos. 6 to 52, are used in optimal reaction conditions. The PCR products are separated by agarose gel electrophoresis and the products are analyzed for the correct identification of *C. glabrata.* The total test time is 3 days.

An alternative method is to take as the patient's blood sample without being seeded by blood culture. In this case, the above procedure is followed and the total test time is 4 hours.

Thus, the critical step is to obtain sufficient genomic DNA from any of the types of samples described above (see obtaining clinical samples section), and from them, using the genomic DNA obtained as the template for PCR where any of the oligonucleotides generated from SEQ ID Nos. 1 to 5, such as but not limited to those described in SEQ ID Nos. 6 to 52, is used under optimal reaction. The PCR products are obtained and analyzed by any conventional method, such as but not limited to agarose gel electrophoresis, dot-blot hybridizations, Southern blotting, Northern blotting and similar RT-PCR, PCR-ELISA, and others known in the art (for example, but not limited to, Molecular Diagnostic PCR handbook. (2005), Gerrit J. Viljoen, Louis H. Nel and John R. Crowther. Springer Publishers) to correctly identify *C. glabrata.* Note that these oligonucleotides may comprise nucleotide unmarked or marked, such as but not limited to, radioactive labeling, chemoluminiscent, luminescent, fluorescent, biotinylated labeling.

To test the utility of the diagnostic method of the present invention, tests with clinical samples from different origins were perfomed, using different combinations of the oligonucleotides generated from SEQ. ID. Nos. 1 to 5. These experiments are described below:
a) Identification of *C. glabrata* in clinical samples of different origins: in Figures 25 and 26 the detection of *C*. *glabrata* is illustrated, in this case, in blood and urine samples. In 100% of the samples, *C. glabrata* was detected, no PCR product was produced in the negative control and samples with *C.albicans.* This indicates that the method of the present invention is 100% sensitive and 100% specific for *C. glabrata* and there is no cross-reaction with other species. The presence or absence of certain bands suggests a possible variation in the serotypes of clinical samples, although more research is needed in this regard. A recent report supporting this hypothesis is in Polakova, S. et al. PNAS, Vol. 106, No. 8, pages 2688-2693, February 24, 2009; in which chromosomal rearrangements in *C. glabrata* are suggested as a virulence mechanism in clinical isolates.Therefore, it is not inconsistent with the object and subject of the present invention the presence or absence of bands in clinical samples, provided that these match in sequence and size to those provided in this application. In addition, to verify the above, by resequencing of the bands obtained, states that both the size and the sequence correspond to the expected PCR products of *C*. *glabrata.*
b) Detection of phylogenetically species related to *C*. *glabrata:* in order to verify that the regions of negative element (NE) and therefore the probes described and claimed in this application are unique to *C. glabrata,* we tested three pairs of oligonucleotides with the following species: *Candida glabrata, Candida albicans, Candida tropicalis, Candida parapsilosis, Candida guillermondii, Candida kruzei, Saccharomyces cerevisiae, Saccharomyces castelii, Saccharomyces bayanus, Saccharomyces kudriavzevii, Saccharomyces mikatae, Saccharomyces parodoxus, Debaryomyces hansenii, Kluyveromyces waltii, Ashby gossypii, Klluyveromyces lactis* and *Yarrowia lipolytica.* As it can be appreciated in Figure 27, the oligonucleotides from SEQ. ID. Nos. 9 and 10 of the present invention only amplified the region of the EN controls for *C. glabrata,* so it is found that EN regions and probes associated are unique to said specie and are useful for the detection of *C. glabrata,* with a sensitivity and specificity of 100%. The bands of Lanes 3 to 17 correspond to the internal control with 26S rRNA probes (lanes 3 to 7) and 18S rRNA (lanes 8 to 17) of the phylogenetically related species listed above.
c) Detection of *C. glabrata* mixed with other phylogenetically close species: in the Figure 28, it is shown the detection of *C. glabrata* in different biological samples and combined with different phylogenetically close species. As can be seen from this figure, there are not PCR products in the lane with samples of *Candida albicans, Candida tropicalis, Candida parapsilosis,* same as they are in a constant amount of 100 ng DNA by tested specie, whereas in samples with a mixture of *C. albicans* and *C. glabrata,* where *C. glabrata* is present in amounts ranging from 345 ng to 2.5 ng, this organism is detected with the PCR products of expected size, but not in the last lane, where the mixture of microorganisms did not included DNA from *C. glabrata.* This is also a testament to the sensitivity and specificity of 100% of the test.

A further embodiment of the present invention comprises a diagnostic kit for *C. glabrata,* which will comprise at least one oligonucleotide generated from SEQ. ID. Nos. 1 to 5, from 15 to 35 pairs of bases, either lyophilized or resuspended in an acceptable carrier, a sample of genomic DNA from *C. glabrata* as positive control and a sample of genomic DNA from a phylogenetically close specie to *C. glabrata* as negative control. Variations in these elements are easily deducible by one skilled in the art and are within the scope of this application.

### Detection method for C. alabrata by nucleic acids hybridization.

Fragments of SEQ. ID Nos. 1 to 5 are useful for the detection of *C*. *glabrata* by methods of conventional nucleic acid hybridization. To this end, the steps are:
a) obtain nucleic acids from a biological sample,
b) hybridizing said nucleic acid from a biological sample with a probe that comes from any continuous fragment of more than 36 base pairs contained in SEQ ID Nos. 1 to 5.
c) detecting the hybridization by a conventional method.

The probe derived from SEQ ID Nos. 1 to 5 can be obtained from restriction enzymes cutting or can be a PCR product. Also, the probe may be radioactively, fluorescence, biotinylation, luminescence, chemiluminescence or the like labeled.

Methods for detecting hybridization of the probe with the biological sample are those known as methods of nucleic acid hybridization and can be selected, for example but not limited to: Dot-Blot, Southern Blot, Northern Blot, microarray, in situ hybridization or the like. As a reference, but not to limit the scope of the invention, is quoted: MOLECULAR BIOMETHODS HANDBOOK 2008 JOHN M. WALKER RALPH RAPLEY, Handbook of Molecular and Cellular Methods in Biology and Medicine, 2nd ed. L. Cseke, et al., Ed., CRC Press, 2004, 600 pp; Ausubel FM et al. (1999) Short protocols in molecular biology: a compendium of Methods from Current protocols in molecular biology. John Wiley & Sons; Fasmann GD (1989) Practical handbook of biochemistry and molecular biology. Florida CRC Press, 1989, Sambrook J, Fritsch EF, Maniatis T (1989) Molecular cloning: a laboratory manual. Cold Spring Harbor Laboratory Press.

A further embodiment of the present invention comprises a diagnostic kit for *C*. *glabrata,* which will comprise at least one molecule DNA molecule of at least 36 base pairs contained within the SEQ. ID. Nos. 1 to 5, either lyophilized or resuspended in an acceptable carrier, a sample of genomic DNA from *C. glabrata* as a positive control and a sample of genomic DNA of a phylogenecally close specie *C. glabrata* as negative control. Variations in these elements are easily deducible by one skilled in the art and are within the scope of this application.

### EXAMPLES

### Example 1. Analysis of clinical urine specimens.

234 clinical urine samples were taken and were classified by the traditional method described above, where 200 samples were classified as not developed fungi, 9 as *C. albicans* and 25 as *Candida sp.* Of these 25 samples, 6 (24%) were classified as *C. glabrata.* With the detection method of the present invention 12 (48%) samples were identified as positive for *C*. *glabrata.*

### Example 2. Comparison between several species of yeast and C. glabrata with the detection method of the present invention.

In Figure 28 it is demonstrated the specificity of the oligonucleotides generated from SEQ. ID. Nos. 1 to 5 in mixtures of *C. glabrata* with phylogenetically close species, such as *C*. *albicans, C. parapsilosis,* and *C*. *tropicalis.* In each of the shown gels oligonucleotides SEQ. ID. Nos. 7 and 8 were used. As can be seen, only in those lanes where *C. glabrata* DNA is present, even mixed with other microorganisms, amplification of fragments is achieved of the expected sizes. Therefore, oligonucleotides of the present invention are capable of specifically detecting the presence of *C. glabrata* in complex samples, even in minimal quantities of *C*. *glabrata* DNA from between 2.5 and 345 ng.

### Example 3. Sensitivity of the diagnostic test of the present invention in clinical isolates.

In order to test the sensitivity of the oligonucleotides generated from SEQ. ID. Nos. 1 through 5 for the identification of *C. glabrata,* clinical samples were tested with oligonucleotides SEQ. ID. Nos. 6 to 10. Figures 29 and 30 show the results of the different amplifications, with the PCR product expected sizes. It should be mentioned that in Figures 29 and 30, lanes 14, 17 and 33 no amplification product was obtained. In these cases, the identification of *C. glabrata* was confirmed with biochemical tests and the results shown in Table 1 were obtained.

**Table 1. Analysis with API-20 test of clinical isolates 14, 17 and 33 of Figures 29 and 30.**

| Clinical isolate | Identified specie | API-20 Code | Description |
|---|---|---|---|
| 14 | Candida paratropicalis | 2552170 | Very good identification. |
| 17 | Candida paratropicalis | 2552170 | Very good identification. |
| 33 | Candida albicans | 2546170 | Acceptable identification of C. albicans |

In addition, PCR products amplified from clinical isolates were re-sequenced to verify that these bands correspond to the expected sequence of *C*. *glabrata* and not to some other species, including humans. In this sequenciation, it was found that indeed the amplified bands in all cases corresponded to the expected specific sequence of *C. glabrata.*

Therefore, through this series of experiments conclusively verify the sensitivity and specificity of the oligonucleotides generated from SEQ. ID. Nos. 1 to 5.

### Example 4. C. glabrata detection using different combinatorial oligonucleotides generated from SEQ ID Nos. 1-5.

In order to verify that any portion of the sequences described in SEQ. ID. Nos. 1 to 5 are useful for identifying *C*. *glabrata,* we generated additional oligonucleotides (SEQ. ID. Nos. 11 to 52), covering all intergenic regions of adhesins EPA1, EPA22, EPAH, EPA6 and EPA7. Also proved its usefulness for both *C. glabrata* alone or in mixtures with other phylogenetically related species. Figures 2 to 11 show different combinatorials obtainable from the oligonucleotides of SEQ ID Nos. 11 to 52 and the expected PCR product sizes. The results show that all of the SEQ. ID. Nos. 1 to 5 serve as the basis for the generation of oligonucleotides specific for *C*. *glabrata,* maintaining sensitivity and specificity of the test, and it is possible to detect the fungus in a mixture with other yeasts. Therefore, any oligonucleotide generated from SEQ. ID. Nos. 1 to 5 falls within the scope of this application.

### Example 5. C. glabrata detection by hybridization of nucleic acids.

To check the efficiency and sensitivity of the detection method by nucleic acid hybridization, a Dot-Blot was performed using as a probe a nucleic acid sequence of 140 bp labeled with common luminescence for SEQ. ID Nos. 1, 2 and 3 to be hybridized against different DNA templates. As shown in Figure 31, said probe hybridazed only against the internal control, DNA from *C. glabrata* from two biological samples from clinical isolates and DNA from a strain of *C. glabrata.* No hybridization was obtained from the DNA of *C*. *albicans* and *S*. *cerevisiae.* Therefore, we find the sensitivity and specificity of the probes derived from SEQ ID Nos. 1 to 5 for the specific 100%identification of *C*. *glabrata* by nucleic acids hybridization methods.

## Claims

1. An oligonucleotide for specific identification of *C*. *glabrata* comprising a nucleic acid having at least 90% sequence identity to one of SEQ ID NOS: 1 to 5 or a complement thereof, which oligonucleotide has between 15 and 35 nucleotides.

2. The oligonucleotide of claim 1, comprising a sequence selected from SEQ ID NOS: 6 to 52.

3. An in vitro method for the specific identification of *C*. *glabrata*, comprising the steps of: a) amplifying DNA fragments from a biological sample by PCR with the oligonucleotides as defined in claims 1 to 2; b) identify the amplified DNA fragments.

4. The method of claim 5, wherein the amplification of DNA fragments is carried out with at least one oligonucleotide as defined in claims 1 or 2.

5. A kit for the specific identification of *Candida glabrata,* comprising at least one oligonucleotide as defined in claims 1 or 2.

6. An in vitro method for the specific identification of *C. glabrata,* comprising the steps of: a) hybridizing DNA fragments from a biological sample with a continuous sequence of 36 or more nucleotides comprised within at least one DNA sequence selected from the group consisting of SEQ ID No. 1, SEQ ID No. 2, SEQ ID No. 3, SEQ ID No. 4 and SEQ ID No. 5; and b) identify the hybridized DNA fragments.

7. The method according to claim 6, wherein steps a) and b) are carried out by a technique selected from the group comprising: Dot-Blot, Southern Blot, Northern Blot, in situ hybridization or microarrays.

8. The method according to claim 6, wherein the biological sample is derived from a subject to study.

## Patentansprüche

1. Oligonukleotid für die spezifische Identifikation von *C*. *glabrata,* umfassend eine Nukleinsäure, die eine Sequenzübereinstimmung von mindestens 90 % mit einer der Sequenz-ID-Nummern 1 bis 5 oder einem Komplement dazu aufweist, wobei das Oligonukleotid zwischen 15 und 35 Nukleotide aufweist.

2. Oligonukleotid nach Anspruch 1, umfassend eine Sequenz, die aus den Sequenz-ID-Nummern 6 bis 52 gewählt ist.

3. In-Vitro-Verfahren für die spezifische Identifikation von C. *glabrata,* umfassend folgende Schritte:
a) Amplifizieren von DNS-Fragmenten einer biologischen Probe durch PCR mit den Oligonukleotiden nach Anspruch 1 bis 2;
b) Identifizieren der amplifizierten DNS-Fragmente.

4. Verfahren nach Anspruch 3, wobei die Amplifikation der DNS-Fragmente mit mindestens einem Oligonukleotid nach Anspruch 1 oder 2 durchgeführt wird.

5. Kit für die spezifische Identifikation von *Candida glabrata,* umfassend mindestens ein Oligonukleotid nach Anspruch 1 oder 2.

6. In-Vitro-Verfahren für die spezifische Identifikation von *C*. *glabrata,* umfassend die folgenden Schritte:
a) Hybridisieren von DNS-Fragmenten einer biologischen Probe mit einer kontinuierlichen Sequenz von 36 oder mehr Nukleotiden, die innerhalb mindestens einer DNS-Sequenz enthalten ist, die aus der Gruppe bestehend aus Sequenz-ID-Nummer 1, Sequenz-ID-Nummer 2, Sequenz-ID-Nummer 3, Sequenz-ID-Nummer 4 und Sequenz-ID-Nummer 5 ausgewählt ist; und
b) Identifizieren der hybridisierten DNS-Fragmente.

7. Verfahren nach Anspruch 6, wobei die Schritte a) und b) durch eine Technik ausgeführt werden, die aus der Gruppe umfassend folgende Techniken ausgewählt ist: Dot-Blot, Southern-Blot, Northern-Blot, In-situ-Hybridisierung oder Microarrays.

8. Verfahren nach Anspruch 6, wobei die biologische Probe von einem zu untersuchenden Subjekt stammt.

## Revendications

1. Un oligonucleotide pour l'identification spécifique de *C*. *glabrata* comprenant un acide nucléique présentant une identité de séquence d'au moins 90% avec une des SEQ ID NOS: 1 à 5 ou un complément de celle-ci, lequel oligonucléotide a entre 15 et 35 nucléotides.

2. L'oligonucléotide selon la revendication 1, comprenant une séquence choisie parmi SEQ ID NOS: 6-52.

3. Un procédé in vitro pour l'identification spécifique de *C*. *glabrata,* comprenant les étapes consistant à : a) amplifier des fragments d'ADN provenant d'un échantillon biologique par PCR avec les oligonucléotides tels que définis dans les revendications 1 à 2 ; b) identifier les fragments d'ADN amplifiés.

4. Le procédé selon la revendication 5, dans lequel l'amplification de fragments d'ADN est effectuée avec au moins un oligonucléotide tel que défini dans les revendications 1 ou 2.

5. Un nécessiare pour l'identification spécifique de *Candida glabrata,* comprenant au moins un oligonucléotide tel que défini dans les revendications 1 ou 2.

6. Un procédé in vitro pour l'identification spécifique de *C*. *glabrata,* comprenant les étapes consistant à : a) hybrider des fragments d'ADN provenant d'un échantillon biologique avec une séquence continue de 36 nucleotides ou plus compris dans au moins une séquence d'ADN choisie dans le groupe constitué de SEQ ID No. 1, SEQ ID No. 2, SEQ ID No. 3, SEQ ID No. 4 et SEQ ID No. 5; et b) identifier les fragments d'ADN hybridés.

7. Le procédé selon la revendication 6, dans lequel les étapes a) et b) sont mises en oeuvre par une technique choisie dans le groupe comprenant : Dot-Blot, Southern Blot, Northern Blot, hybridation in situ ou microréseaux.

8. Le procédé selon la revendication 6, dans lequel l'échantillon biologique est dérivé d'un sujet à étudier.
